# EUROPEAN PATENT SPECIFICATION

(11) **EP 2 074 928 B1**
(45) Date of publication and mention of the grant of the patent: **29.10.2014**
(21) Application number: 08172717.4
(22) Date of filing: 23.12.2008
(51) Int. Cl.: A61B 1/00, A61B 1/018, G02B 23/24, G02B 23/26, A61B 1/04

(54) **Observation unit detachable type endoscope and endoscope main body**
Endoskop mit abnehmbarer Beobachtungseinheit und Endoskophauptkörper
Unité d'observation, endoscope de type détachable et corps principal d'endoscope

(30) Priority: 27.12.2007 JP 2007338342; 31.12.2007 US 9704 P; 17.12.2008 JP 2008320634
(43) Date of publication of application: 01.07.2009
(73) Proprietor: Olympus Corp., Shibuya-ku Tokyo 151-0072 (JP)
(72) Inventor: Iede, Taro, Tokyo 192-8512 (JP); Tanaka, Hirokazu, Tokyo 192-8512 (JP); Ito, Yoshiaki, Tokyo 192-8512 (JP); Kitagawa, Hideya, Tokyo 192-8512 (JP); Tamura, Hajime, Tokyo 192-8512 (JP)
(74) Representative: Winter, Brandl, Fürniss, Hübner, Röss, Kaiser, Polte - Partnerschaft mbB

(56) References cited:
- EP-A1- 1 955 643
- EP-A1- 2 074 929
- US-A- 4 809 678
- US-A- 4 991 564

## Description

The present invention relates to an endoscope with detachable observation unit for operating on a part in a body cavity, and a main body of the endoscope.

An endoscopic surgical apparatus is known which inserts an endoscope and a surgical instrument introduction tool into a body cavity by means of an over-tube to operate on an affected part in the body cavity with a surgical instrument introduced into the body cavity by the surgical instrument introduction tool while observing the inside of the body cavity with the endoscope (Jpn. Pat. Appln. KOKAI Publication No. 2000-325303). In this endoscopic surgical apparatus, the endoscope including a camera section and a bending mechanism and the surgical instrument introduction tool including a bending mechanism are used in a state in which they are inserted in the common over-tube. Moreover, each of the endoscope and the surgical instrument introduction tool includes the bending mechanism protruding from the distal end of the over-tube so that the respective bending mechanisms individually move. Furthermore, the endoscope and the surgical instrument introduction tool are operated and bent independently of each other.

On the other hand, an endoscope including an operating arm section provided at the distal end of an insertion section provided with a bending portion is disclosed in Jpn. Pat. Appln. KOKAI Publication No. 2005-095590.

The endoscopic surgical apparatus of Jpn. Pat. Appln. KOKAI Publication No. 2000-325303 mentioned above has a configuration for introducing the endoscope and the surgical instrument introduction tool into the body cavity by means of the over-tube, and hence the endoscope and the surgical instrument introduction tool are individually and independently operated and bent in the body cavity. Therefore, it is difficult to cooperatively operate the endoscope and the surgical instrument introduction tool, and a large burden is imposed on an operator who accesses a target part in the body cavity to perform a surgical procedure.

Moreover, in the endoscope disclosed in Jpn. Pat. Appln. KOKAI Publication No. 2005-095590, since the operating arm section is provided at the distal end of the insertion section, a constitution becomes complicated as compared with a usual endoscope for observation. Therefore, enormous labor and time are required for cleaning/disinfection/sterilization, and an operation cost per surgical procedure disadvantageously mounts.

EP 1 955 643 A1 discloses an endoscope according to the preamble of the independent claim 1.

EP 2 074 929 A1 claiming the same priority date as the present invention also discloses an endoscope main body and an endoscope.

An object of the present invention is to provide, as a system in which an observation unit is attachable to/detachable from an endoscope main body, an endoscope with detachable observation unit which decreases burdens imposed on an operator to improve cleaning properties and a main body of the endoscope.

According to the present invention, there is provided an endoscope main body of an endoscope with detachable observation unit according to claim 1. Advantageous embodiments are subject of the subclaims.

The invention can be more fully understood from the following detailed description when taken in conjunction with the accompanying drawings, in which:
FIG. 1 is a perspective view schematically showing the whole operative endoscope according to a first embodiment not according to the claimed invention;
FIG. 2A is a schematic explanatory view of the whole operative endoscope according to the first embodiment;
FIG. 2B is a top plan view showing the distal end of a body cavity insertion section of the operative endoscope from the direction of an arrow 2A shown in FIG. 2A;
FIG. 3A is a vertical sectional view of an insertion section main body of an endoscope main body of the operative endoscope according to the first embodiment;
FIG. 3B is a vertical sectional view showing that an observation unit is attached to the insertion section main body of the endoscope main body of the operative endoscope according to the first embodiment;
FIG. 4 is a vertical sectional view showing a flexible portion and a bending portion in the insertion section main body of the operative endoscope according to the first embodiment;
FIG. 5 is a top longitudinal sectional view of the distal end portion of the insertion section main body of the operative endoscope according to the first embodiment;
FIG. 6 is a front view of the distal end portion of the insertion section of the operative endoscope according to the first embodiment;
FIG. 7 is a cross-sectional view along line A-A of FIG. 3B;
FIG. 8 is a cross-sectional view along line B-B of FIG. 3B;
FIG. 9 is a plan view of a branching member of the endoscope main body of the operative endoscope according to the first embodiment;
FIG. 10 is an explanatory view of a portion of a separation plate in the branching member of the operative endoscope according to the first embodiment;
FIG. 11 is a cross-sectional view of the flexible portion in the insertion section main body of the operative endoscope according to the first embodiment;
FIG. 12A is a side view of a camera section unit in the observation unit attachable to/detachable from the endoscope main body of the operative endoscope according to the first embodiment;
FIG. 12B is a front view of the camera section unit of the observation unit;
FIG. 12C is a plan view of the camera section unit of the observation unit;
FIG. 13A is a vertical sectional view of the camera section unit in the observation unit attachable to/detachable from the endoscope main body of the operative endoscope according to the first embodiment;
FIG. 13B is a cross-sectional view along line C-C of FIG. 13A;
FIG. 14 is a cross-sectional view of a flexible portion of an insertion section main body of an operative endoscope according to a second embodiment corresponding to the invention;
FIG. 15 is a cross-sectional view of a flexible portion of an insertion section main body of an operative endoscope according to a third embodiment corresponding to the invention;
FIG. 16 is a cross-sectional view of a flexible portion of an insertion section main body of an operative endoscope according to a first example helpful for understanding the invention but not part of the invention;
FIG. 17 is a cross-sectional view of a flexible portion of an insertion section main body of an operative endoscope according to a second example;
FIG. 18 is an explanatory view of a binding member of the operative endoscope shown in FIG. 17 according to the second example;
FIG. 19 is a cross-sectional view of a flexible portion of an insertion section main body of an operative endoscope according to a third example;
FIG. 20 is a perspective view of a binding member of the operative endoscope shown in FIG. 19 according to the third example;
FIG. 21 is a cross-sectional view of a flexible portion of an insertion section main body of an operative endoscope according to a fourth example;
FIG. 22 is a cross-sectional view of a flexible portion of an insertion section main body of an operative endoscope according to a fourth embodiment;
FIG. 23 is a cross-sectional view of a flexible portion of an insertion section main body of an operative endoscope according to a fifth embodiment;
FIG. 24 is a vertical sectional view of a portion around a camera section unit in an observation unit according to a sixth embodiment;
FIG. 25 is a vertical sectional view of a portion around a camera section unit in an observation unit according to an seventh embodiment;
FIG. 26 is a cross-sectional view of a portion around a cable unit in the observation unit of FIG. 25;
FIG. 27 is a vertical sectional view of a portion around a camera section unit in an observation unit according to an eight embodiment;
FIG. 28 is a cross-sectional view of a portion around a cable unit in an observation unit according to a ninth embodiment;
FIG. 29A is an explanatory view showing an operative endoscope according to a tenth embodiment;
FIG. 29B is an explanatory view showing the operative endoscope according to the tenth embodiment;
FIG. 29C is a schematic perspective view showing an enlarged operating section of the operative endoscope according to the tenth embodiment;
FIG. 30 is a front view of the distal end portion of an insertion section of an endoscope main body of an operative endoscope according to an eleventh embodiment;
FIG. 31 is a vertical sectional view of the distal end portion of the insertion section of the endoscope main body of the operative endoscope along line D-D of FIG. 30;
FIG. 32 is a perspective view of a holding member which supports a light guide of the endoscope main body in the operative endoscope according to the eleventh embodiment;
FIG. 33 is an explanatory view showing a state before incorporating a camera section unit in a second rigid portion of the endoscope main body of the operative endoscope according to the eleventh embodiment;
FIG. 34 is an explanatory view of a state in which the camera section unit is incorporated in the second rigid portion of the endoscope main body of the operative endoscope according to the eleventh embodiment; and
FIG. 35 is a vertical sectional view of the state in which the camera section unit is incorporated in the second rigid portion of the endoscope main body of the operative endoscope according to the eleventh embodiment.

The best mode for carrying out this invention will hereinafter be described with reference to the drawings.

### [First Embodiment]

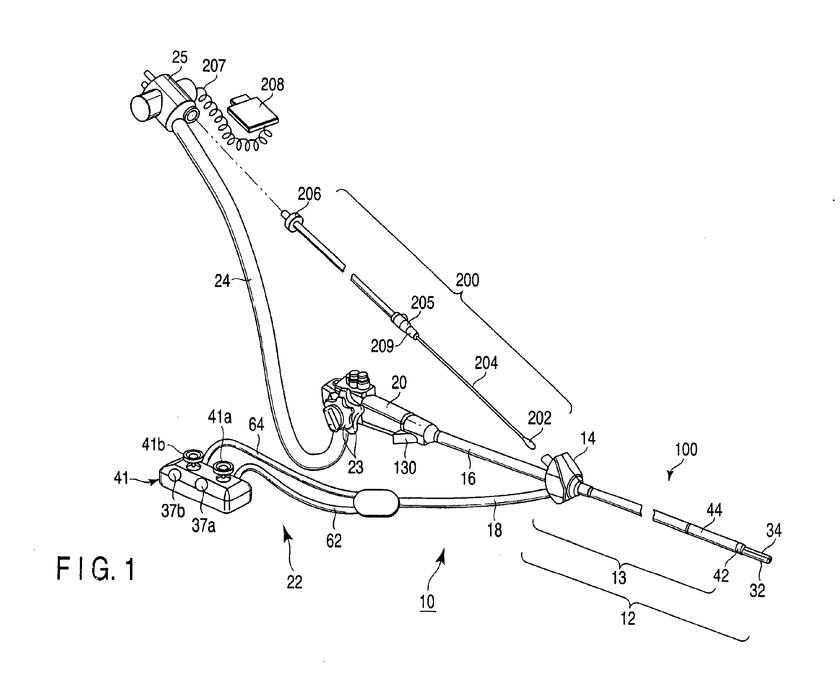

A first embodiment not according to the claimed invention, will be described with reference to FIGS. 1 to 13B. As shown in FIGS. 1 to 2B, an observation unit separation type operative endoscope 10 according to this embodiment includes an endoscope main body 100 and an observation unit (an observation optical system) 200.

The endoscope main body 100 includes a body cavity insertion section 12 which is inserted into a body cavity; a branching member (a branching section) 14 arranged at the proximal end portion of the body cavity insertion section 12; a first extending section 16 and a second extending section 18 which branch from the proximal end side of the branching member 14 and separately extend rearwards; a first operating section (a main body operating section) 20 arranged at the proximal end portion of the first extending section 16; a second operating section (an arm section operating section) 22 arranged at the proximal end portion of the second extending section 18; and a universal cord 24 extending from the first operating section 20.

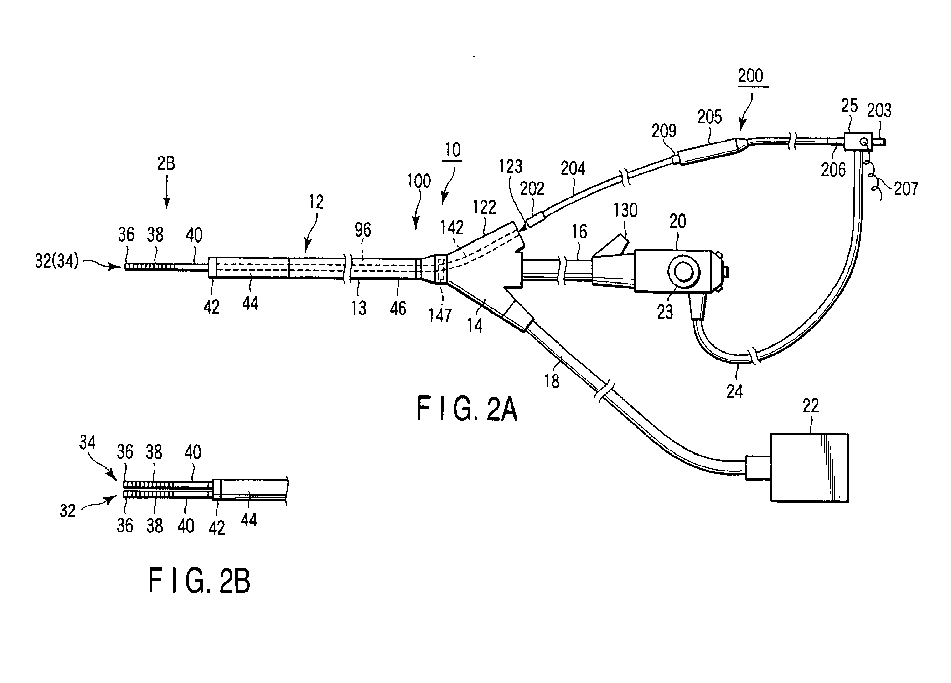

Each of the first extending section 16 and the second extending section 18 is formed of a long member having flexibility. The first operating section 20 has a bending operation knob (a handle) 23 as an operation mechanism (a first operation mechanism) for operating and bending a third bending portion (a main body bending portion) 44 described later. When the bending operation knob 23 is operated, the third bending portion (main body bending portion) 44 in the body cavity insertion section 12 described later is bent. A main body member of the first operating section 20 is provided with an insertion opening 130 as an inlet from which a surgical instrument (not shown) such as a pair of forceps is inserted into a third channel tube 66 described later. As shown in FIG. 2A, the insertion opening 130 is formed in such a manner that the opening obliquely extends rearwards to face a side deviating from a central axis of the first extending section 16.

The second operating section 22 is provided with an operation unit (an arm section operating section) 41 as an operation mechanism (a second operation mechanism) for operating and bending an arm section for an arm manipulation for a surgical procedure described later. As shown in FIG. 1, the operation unit 41 includes an operation handle 41a which operates and bends a first bending portion 38 of a first arm section 32 as the arm section for the arm manipulation for the surgical procedure, and an operation handle 41 b which operates and bends a second bending portion 40 of the first arm section 32 as the arm section for the arm manipulation for the surgical procedure. One bending portion may be incorporated in the first arm section 32 and a second arm section 34, but here, as shown in FIG. 2B, each of the first arm section 32 and the second arm section 34 includes two bending portions, that is, the first bending portion 38 and the second bending portion 40. Therefore, the respective operation handles 41a, 41b can individually operate the respective bending portions 38, 40 of the respective arm sections 32, 34.

As shown in FIG. 1, a main body of the operation unit 41 is provided with a first insertion port 37a communicating with a first channel tube 62 which guides a surgical instrument to the first arm section 32, and a second insertion port 37b communicating with a second channel tube 64 which guides another surgical instrument to the second arm section 34. The first insertion port 37a is a forceps opening into which the surgical instrument to be guided to the first arm section 32 is inserted. The second insertion port 37b is a forceps opening into which the surgical instrument to be guided to the second arm section 34 is inserted. Moreover, the channel tubes 62, 64 can be utilized not only for inserting or withdrawing the surgical instruments but also for supplying or draining water or injecting a reagent from the respective arm sections 32, 34 corresponding to the channel tubes into the body cavity.

Meanwhile, during a manual procedure for operation on living tissue by means of the operative endoscope 10, for example, an operation of rotating the proximal end portion of the body cavity insertion section 12 or the first extending section 16 around an axis thereof (a twisting operation) is sometimes performed. Therefore, the first extending section 16 and the first operating section 20 are arranged along the same axis as a central axis extending from the branching member 14 through the body cavity insertion section 12. In consequence, when the above twisting operation is performed, an operation force for rotating the proximal end portion of the body cavity insertion section 12 or the first extending section 16 around the axis thereof can be readily transmitted to the distal end side of the body cavity insertion section 12 as compared with a case where the first extending section and the first operating section are not coaxially arranged, and operability when using the operative endoscope is improved.

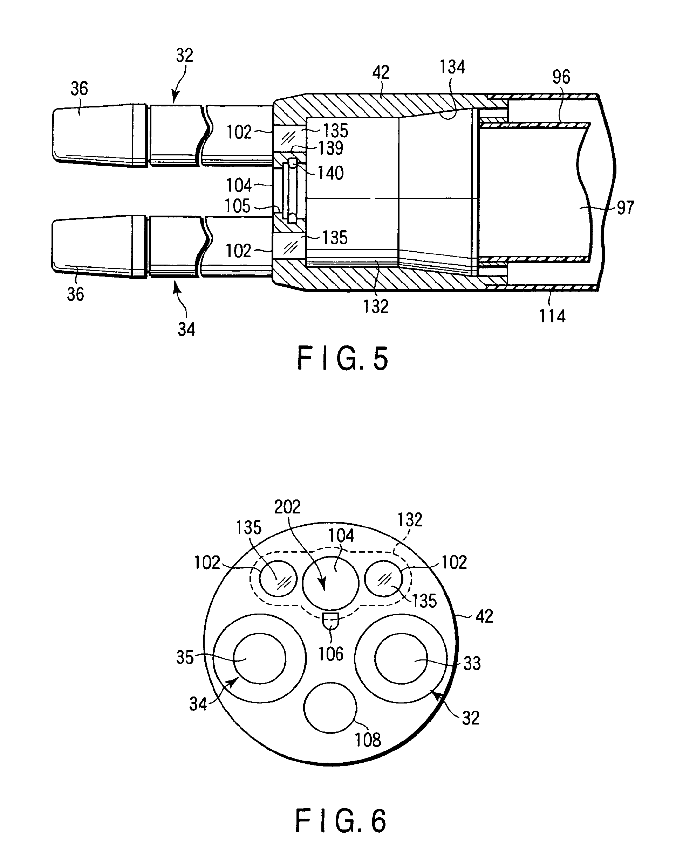

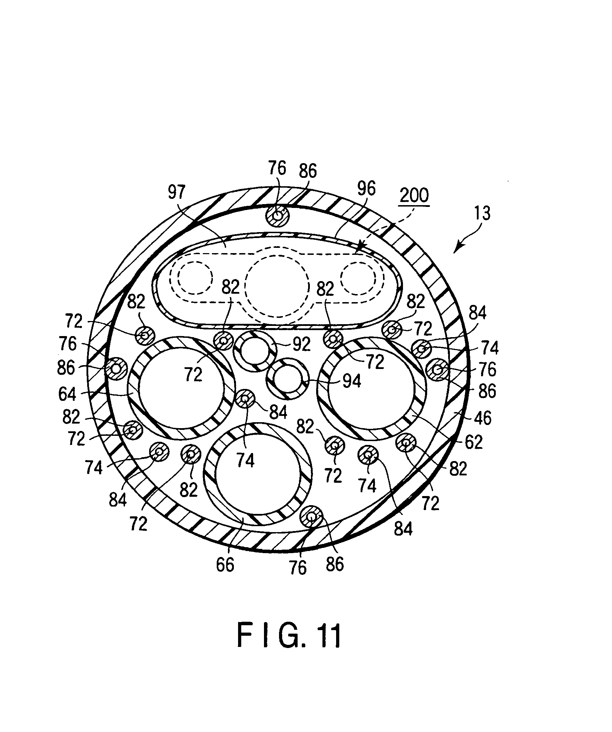

As shown in FIGS. 1 and 2A, the body cavity insertion section 12 includes an insertion section main body 13 which serves as a main body of the insertion section, and one or more operating arm sections 32, 34 whose proximal ends are connected to the distal end of the insertion section main body 13 and which protrude forwards. In this configuration, the body cavity insertion section is a bi-arm section including the first arm section 32 and the second arm section 34 arranged on left and right sides. The first arm section 32 includes a first rigid portion (a distal end portion) 36 positioned at the most distal end of the insertion section 12, the first bending portion (arm section bending portion) 38 connected to the proximal end of the first rigid portion 36, and the second bending portion (arm section bending portion) 40 connected to the proximal end of the first bending portion 38. The proximal end of the second bending portion 40 is fixedly connected to the distal end of the insertion section main body 13. The second arm section 34 also includes a first rigid portion (a distal end portion) 36 positioned at the distal end, the first bending portion (arm section bending portion) 38 connected to the proximal end of the first rigid portion 36, and the second bending portion (arm section bending portion) 40 connected to the proximal end of the first bending portion 38 in the same manner as in the first arm section 32. The proximal end of the second bending portion 40 is fixedly connected to the distal end of the insertion section main body 13. The first arm section 32 and the second arm section 34 are provided with channels 33, 35, respectively (see FIG. 6). As shown in FIG. 11, the channel 33 of the first arm section 32 is connected to the first channel tube 62, and the channel 35 of the second arm section 34 is connected to the second channel tube 64.

As shown in FIG. 2A, the insertion section main body 13 includes a second rigid portion (a distal end portion) 42 positioned at the most distal end of the insertion section main body 13, the third bending portion (main body bending portion) 44 connected to the proximal end of the second rigid portion 42, and a flexible portion (a corrugated tube portion) 46 connected to the proximal end of the third bending portion 44. The proximal end of the first arm section 32 and the proximal end of the second arm section 34 are both connected to the distal end of the insertion section main body 13. That is, the proximal ends are supported by the distal end of the second rigid portion 42 of the insertion section main body 13. Therefore, when the third bending portion 44 bends, the first arm section 32 and the second arm section 34 follow the bending of the third bending portion 44 to move together with the distal end of the insertion section main body 13. That is, the first arm section 32 and the second arm section 34 as arms for the surgical procedure follow the bending of the third bending portion 44 to move based on the position of the distal end of the third bending portion 44. Therefore, when the third bending portion 44 is bent to move the distal end of the insertion section main body 13, the first arm section 32 and the second arm section 34 move following the distal end of the insertion section main body 13. Therefore, the states of the first arm section 32 and the second arm section 34 can be observed in a view field of the observation unit 200 arranged at the distal end of the second rigid portion 42. Since the first arm section 32 and the second arm section 34 move following the distal end of the insertion section main body 13, the first arm section 32 and the second arm section 34 hardly go out of sight. Even if the arm sections go out of sight, the arm sections can easily be returned to the view field. Therefore, the surgical procedure by the first arm section 32 and the second arm section 34 can easily and further quickly be performed.

Here, although each arm section 32 or 34 includes a plurality of bending portions, that is, the first bending portion 38 and the second bending portion 40, it may include, for example, only one bending portion or three or more bending portions. Moreover, an indirect support member such as a flexible tube (a corrugated tube) may be arranged between the first bending portion 38 and the second bending portion 40 or between the second bending portion 40 and the second rigid portion 42 at the distal end of the insertion section main body 13. Here, although not described in detail, the first bending portion 38 and the second bending portion 40 may have a structure similar to that of the third bending portion 44.

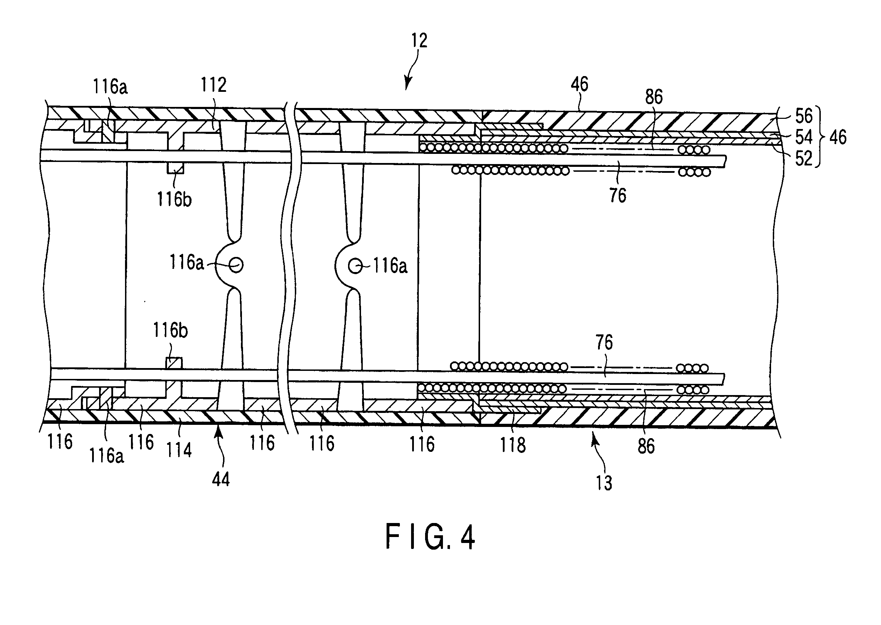

Next, a structure of the insertion section main body 13 will specifically be described. As shown in FIG. 4, the flexible portion 46 of the insertion section main body 13 includes a cylindrically formed helical tube 52, a mesh-like blade 54 arranged on the outer side of the helical tube 52, and an outer tube 56 arranged on the outer side of the blade 54. The helical tube 52 is formed into a substantially cylindrical shape by spirally winding, for example, a thin-film material of stainless steel. The blade 54 is formed into a substantially cylindrical shape by combining wire bundles each obtained by bundling, for example, a plurality of stainless steel wires. The outer tube 56 is formed into a substantially cylindrical shape to cover the outer side of the blade 54 with a polymer material having flexibility, for example, a rubber material, a resin material or the like.

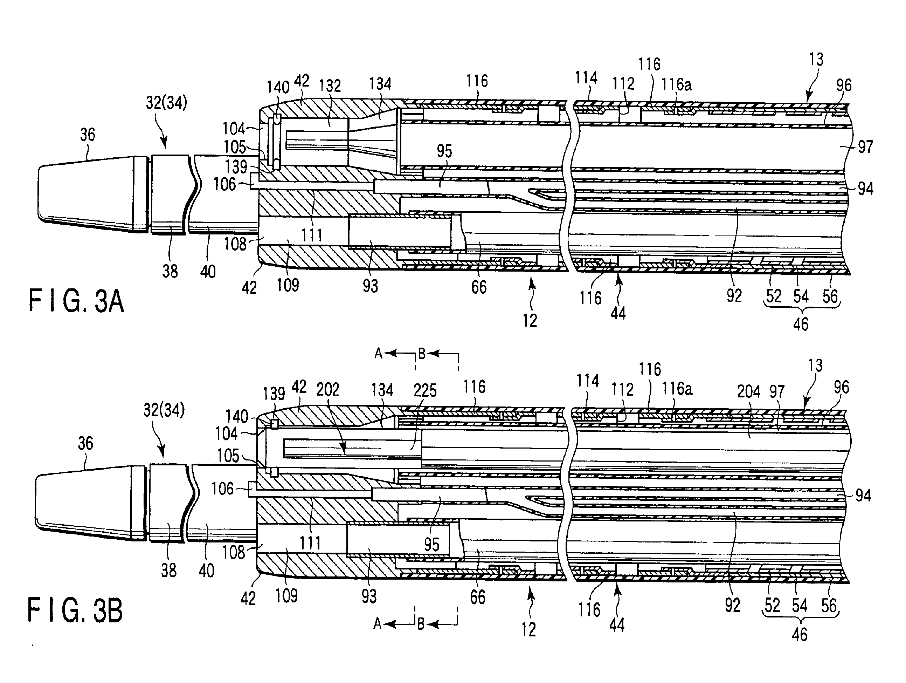

As shown in FIGS. 3A to 4, the third bending portion 44 positioned on the distal end side of the flexible portion 46 includes a bending tube 112 constituted of a tubular member which can bend in, for example, four directions, that is, upper, lower, left and right directions, and an outer tube 114 which covers the bending tube 112. The outer tube 114 includes a blade arranged on the inner surface thereof. The bending tube 112 has a plurality of bending pieces 116, the plurality of bending pieces 116 are arranged in one row in the axial direction of the third bending portion 44, and the adjacent front and rear bending pieces 116 are rotatably connected to each other via a pin 116a. As shown in FIGS. 3A and 3B, the portion of the bending tube 112 positioned at the most distal end is fixed to the second rigid portion 42 by, for example, an adhesive, screws or the like. As shown in FIG. 4, a connection mouth ring 118 is arranged between the proximal end of the bending tube 112 and the distal end of the flexible portion 46. The connection mouth ring 118 connects the proximal end of the third bending portion 44 to the distal end of the flexible portion 46.

As shown in FIG. 4, for example, densely wound coil-like third wire guides 86 are divided and arranged in four positions, that is, upper, lower, left and right positions equally separated from one another on the inner peripheral surface of the connection mouth ring 118, and the distal ends of the respective third wire guides 86 are fixed to the connection mouth ring 118. Third wires 76 for bending the third bending portion 44 are separately inserted through the respective third wire guides 86. The distal end portions of the third wires 76 independently extend from the distal ends of the third wire guides 86 to the third bending portion 44. The inner peripheral surfaces of the bending pieces 116 are provided with wire receiving portions 116b for guiding the third wires 76. The distal end of each third wire 76 is fixed to the distal end of the bending tube 112 (bending piece 116 placed at the most distal end) or the second rigid portion 42. Therefore, when each of the upper, lower, left and right third wires 76 is pulled toward the front side in the axial direction, the third bending portion 44 bends in its pulling direction. Although not shown in this embodiment, a mesh-like blade such as the blade 54 of the flexible portion 46 may be arranged between the bending tube 112 and the outer tube 114.

As shown in FIG. 11, in the flexible portion 46 of the insertion section main body 13, a plurality of later-described internal members are arranged, and a guide tube (a tubular member) 96 for guiding the observation unit 200 is arranged while avoiding these internal members. It is to be noted that examples of the internal members mentioned herein include first to third channel tubes 62, 64 and 66, four pairs of first wires 72, two pairs of second wires 74, two pairs of third wires 76, first wire guides 82, second wire guides 84 and third wire guides 86 which separately cover these wires 72, 74 and 76, respectively, an air supply tube 92 and a water supply tube 94, but they are not restrictive.

Although the guide tube 96 is also one of the internal members, the guide tube 96 is a separation member in regard to points that an insertion path (a path) 97 for guiding the observation unit 200 is formed as described later and that the guide tube 96 itself is separated from the other internal members to form the insertion path 97 separately from the other internal members. In these points, the guide tube 96 is distinguished from the other internal members.

As shown in FIG. 11, an observation unit attachment/detachment mechanism for detachably attaching the observation unit 200 to the insertion section main body 13 is incorporated in the insertion section main body 13. The observation unit attachment/detachment mechanism includes the guide tube (guide tube for the observation unit) 96 arranged in the insertion section main body 13 while avoiding the above internal members. Here, the guide tube 96 is formed as a tubular member having a substantially flat cross-sectional shape. Moreover, the guide tube 96 has the substantially flat cross-sectional shape, and hence constitutes a regulating portion which determines the direction of the observation unit 200 inserted into the guide tube 96 around the axis of the observation unit 200. Therefore, here, the guide tube 96 itself constitutes an insertion guide mechanism of the observation unit 200 including the insertion path (path) 97 which guides the observation unit 200 therethrough and which is separated from the other internal members, and simultaneously constitutes the observation unit attachment/detachment mechanism.

The first channel tube 62 shown in FIG. 11 is led to the first rigid portion 36 of the first arm section 32 from the flexible portion 46 of the insertion section main body 13 through the third bending portion 44 and the second rigid portion 42, to form a passage reaching the opening of a hole formed in the first rigid portion 36. That is, the distal end of the first channel tube 62 is connected to the channel 33 formed in the first arm section 32. The proximal end portion of the first channel tube 62 is connected to the first insertion port 37a of the second operating section 22 from the second rigid portion 42 through the third bending portion 44, the flexible portion 46, the branching member 14 and the second extending section 18.

Like the first channel tube 62, the second channel tube 64 shown in FIG. 11 is led to the first rigid portion 36 of the second arm section 34 from the flexible portion 46 through the third bending portion 44 and the second rigid portion 42. The distal end of the second channel tube 64 forms a passage reaching the opening of a hole formed in the first rigid portion 36. That is, the second channel tube 64 communicates with the channel 35 formed in the second arm section 34. The proximal end side of the second channel tube 64 is connected to the second insertion port 37b of the second operating section 22 from the second rigid portion 42 through the third bending portion 44, the flexible portion 46, the branching member 14 and the second extending section 18.

On the other hand, as shown in FIGS. 3A, 3B and 11, the distal end of the third channel tube 66 is connected to a distal end opening (a forceps opening) 108 formed in the second rigid portion 42 of the insertion section main body 13. The distal end of the third channel tube 66 is connected to a connection tube 93 fixed to the second rigid portion 42. As shown in FIGS. 3A and 3B, the connection tube 93 is connected to a connection hole 109 which forms the distal end opening 108 of the third channel. The proximal end side of the third channel tube 66 is led through the third bending portion 44, the flexible portion 46, the branching member 14 and the first extending section 16, and connected to the insertion opening (forceps opening) 130 of the first operating section 20.

Each of the first bending portions 38 of the first arm section 32 and the second arm section 34 is operated and bent in the four directions, that is, upper, lower, left and right directions by two pairs of first wires (bending operation wires) 72. The distal ends of the two pairs of first wires 72 are connected to the corresponding first bending portions 38 of the first arm section 32 and the second arm section 34, respectively. Each of the proximal end portions of the two pairs of the first wires 72 is led to the second operating section 22 through the second bending portion 40, the second rigid portion 42, the third bending portion 44, the flexible portion 46, the branching member 14 and the second extending section 18 along the outer periphery of the channel tube 62 or 64 corresponding to the arm section 32 or 34 of the corresponding first bending portion 38 to be operated and bent by the first wire 72.

Each of the second bending portions 40 of the first arm section 32 and the second arm section 34 is operated and bent in two left and right directions by one pair of second wires (bending operation wires) 74. The distal ends of the pair of second wires (bending operation wires) 74 are connected to the corresponding second bending portions 40 of the first arm section 32 and the second arm section 34, respectively. The proximal end side of each second wire 74 is connected to the second operating section 22 through the second rigid portion 42, the third bending portion 44, the flexible portion 46, the branching member 14 and the second extending section 18 along the outer periphery of the channel tube 62 or 64 corresponding to the arm section of the corresponding second bending portion 40 to be operated and bent by the second wire 74.

Moreover, when the second operating section 22 is used to axially move each pair of first wires 72, the corresponding first bending portions 38 of the arm sections 32, 34 are bent in, for example, four directions, that is, upper, lower, left and right directions, respectively..When the second operating section 22 is used to axially move each pair of second wires 74, the corresponding second bending portions 40 of the arm sections 32, 34 are bent in, for example, two directions, that is, left and right directions, respectively. The first bending portion 38 of the first arm section 32 and the first bending portion 38 of the second arm section 34 can be bent independently of each other. The second bending portion 40 of the first arm section 32 and the second bending portion 40 of the second arm section 34 can be bent independently of each other. It is to be noted that the first bending portion 38 and the second bending portion 40 may be bent not only in the above directions but also in another direction.

The distal ends of the first wire guides 82 through which the first wires 72 are individually inserted and guided are connected to the corresponding proximal ends of the first bending portions 38 of the first and second arm sections 32, 34, respectively. Each of the proximal end portions of the first wire guides 82 is led into the second operating section 22 from the second extending section 18 through the second bending portion 40, the second rigid portion 42, the third bending portion 44, the flexible portion 46 and the branching member 14 along the outer periphery of the channel tube 62 or 64 corresponding to the arm section 32 or 34 having the first bending portion 38 as a target to be operated and bent by the first wire 72. The proximal end portions of the first wire guides 82 do not necessarily have to be fixed.

The distal ends of the second wire guides 84 through which the second wires 74 are individually inserted and guided are connected to the corresponding proximal ends of the second bending portions 40 of the first and second arm sections 32, 34, respectively. Each of the proximal end portions of the second wire guides 84 is led into the second operating section 22 from the second extending section 18 through the second rigid portion 42, the third bending portion 44, the flexible portion 46 and the branching member 14 along the outer periphery of the channel tube 62 or 64 corresponding to the arm section 32 or 34 having the second bending portion 40 as a target to be operated and bent by the second wire 74. The proximal end portions of the second wire guides 84 do not necessarily have to be fixed.

On the other hand, the distal ends of two pairs of third wires 76 for operating and bending the third bending portion (main body bending portion) 44 of the body cavity insertion section 12 are connected to members around the distal end of the third bending portion 44 by brazing, soldering or the like. Each of the proximal end portions of the third wires 76 is led to the first operating section 20 from the insertion section main body 13 through the flexible portion 46, the branching member 14 and the first extending section 16. The distal ends of two pairs of third wire guides 86 which guide the third wires 76 are connected to the connection mouth ring 118 shown in FIG. 4. Each of the proximal end portions of the third wire guides 86 is led into the first operating section 20 through the flexible portion 46, the branching member 14 and the first extending section 16. The first to third wire guides 82, 84 and 86 are formed of, for example, densely wound coil-like tube members, respectively. The proximal end portions of the third wire guides 86 do not necessarily have to be fixed.

Therefore, when two pairs of third wires 76 are axially moved by the first operating section 20, respectively, the third bending portion 44 is bent in the corresponding direction. In the present embodiment, since the two pairs of third wires 76 are disposed, the third bending portion 44 can be bent in, for example, four directions, that is, upper, lower, left and right directions.

The distal ends of the air supply tube 92 and the water supply tube 94 shown in FIG. 11 are united and connected to a connection tube 95 fixed to the second rigid portion 42 near the second rigid portion 42 of the insertion section main body 13 shown in FIGS. 3A and 3B. In consequence, the air supply tube 92 and the water supply tube 94 communicate with a common nozzle 106 through a connection hole 111 provided in the second rigid portion 42. Each of the proximal end portions of the air supply tube 92 and the water supply tube 94 is led to a connector 25 provided at the end of the universal cord 24 through the third bending portion 44, the flexible portion 46, the branching member 14, the first extending section 16 and the first operating section 20 and further through the universal cord 24 (see FIG. 2A).

As shown in FIGS. 3A and 3B, the guide tube 96 for the observation unit 200 is arranged in a determined region of a space in the insertion section main body 13 of the body cavity insertion section 12. That is, the guide tube 96 for the observation unit 200 is arranged through the predetermined region from a position regulated by a later-described separation plate 147 provided in the branching member 14 shown in FIGS. 2A, 9 and 10 to an observation opening (an observation window) 104 of the second rigid portion 42 constituting the distal end portion of the insertion section main body 13. The proximal end portion of the guide tube 96 is fixed to a later-described guide member (observation unit attachment/detachment mechanism) 142 through the predetermined region of the separation plate 147. Moreover, the distal end of the guide tube 96 is arranged at a predetermined position of the second rigid portion 42. The guide tube 96 defines a region separated from the other internal members in the inner region of the insertion section main body 13, to form the insertion path (path) 97 as an observation unit guide mechanism which guides the observation unit 200 to the distal end of the body cavity insertion section 12 as described later.

The guide tube 96 is formed as a tube having a substantially flat cross-sectional shape shown in FIG. 11 using a resin such as polytetrafluoroethylene (4-ethylene fluoride) or a tetrafluoroethylene/hexafluoropropylene copolymer (4.6-ethylene fluoride), a metal foil of aluminum, or any other material.

The guide tube 96 has such flexibility as to follow deformation of the flexible portion 46 and the third bending portion 44 of the insertion section main body 13 to such an extent that a function of guiding the observation unit 200 is not impaired. Therefore, the guide tube 96 has such flexibility and elasticity as to expand in accordance with the thickness or the shape of the observation unit 200 when guiding the observation unit 200. In consequence, even in a case where the guide tube is inserted through the observation unit 200 in which a cable unit 204 is thinner than a camera section unit 202 incorporated in the observation unit 200, a portion of the guide tube 96 passed through the camera section unit 202 and then the cable unit 204 contracts, or the guide tube 96 itself can readily bend. Therefore, the deformation of the flexible portion 46 and the bending portion 44 of the insertion section main body 13 is not noticeably disturbed.

Next, a structure of the distal end portion of the body cavity insertion section 12 will be described. As shown in FIGS. 5 and 6, in the distal end surface of the second rigid portion 42 are formed a pair of illumination windows 102, the observation opening 104 in which the distal end of the camera section unit 202 of the observation unit 200 described later is positioned when arranged, an air/water supply nozzle 106 having a jet port directed to the distal end surface of the camera section unit 202 attached to the observation opening 104, and the distal end opening (channel port) 108 connected to the third channel tube 66 so that they are exposed. Each illumination window 102 includes a distal end lens 135 which also serves as a transparent cover. The observation opening 104 is formed as an opening hole reaching the outside. Here, the observation opening 104 is formed and opened, but a portion of the observation opening 104 may be closed with a transparent cover.

As shown in FIGS. 5 and 6, the observation opening 104 is arranged between the pair of illumination windows 102. In a region positioned below the pair of illumination windows 102 and the observation opening 104, the first arm section 32 and the second arm section 34 arranged on the left and right sides of the observation opening 104 are substantially laterally symmetrically provided. The air/water supply nozzle 106 is provided right under the observation opening 104. The distal end opening 108 is arranged in a region below the air/water supply nozzle 106. The observation opening 104, the air/water supply nozzle 106 and the distal end opening 108 are substantially arranged in one row while the centers thereof are positioned along a vertical line passing through the center of the distal end surface of the second rigid portion 42. The pair of illumination windows 102 are arranged laterally symmetrically with respect to the observation opening 104. The first arm section 32 and the second arm section 34 are arranged below the pair of illumination windows 102 and the observation opening 104 and arranged substantially laterally symmetrically with respect to the observation opening 104. Consequently, postures or movements of the first arm section 32 and the second arm section 34 can easily be observed by the observation unit 200. Here, the first arm section 32 and the second arm section 34 are arranged below the observation opening 104, but the arrangement may vertically be inverted, and there is not any special restriction on this arrangement relation.

The second rigid portion 42 shown in FIGS. 3A, 3B and 5 is formed into a substantially columnar shape using a metal material such as a stainless steel material, or a hard resin material. When the second rigid portion 42 is made of the metal material, the outer periphery thereof is covered with an insulating material. In the second rigid portion 42, one concave space is positioned on the inner sides of the two illumination windows 102 and the observation opening (observation window) 104 so as to communicate with both of them, and has a size corresponding to combined inner regions of both the two illumination windows 102 and the observation opening 104. The concave space forms a receiving chamber (observation unit attachment/detachment mechanism) 132 in which the camera section unit 202 constituting the distal end portion of the observation unit 200 described later is positioned in a predetermined direction and received. The receiving chamber 132 has a shape and a size such that the cross-sectional shape of the receiving chamber is adapted to that of the camera section unit 202 described later, and hence the receiving chamber 132 is formed so that the camera section unit 202 received in the receiving chamber 132 is positioned in a predetermined direction. That is, the receiving chamber 132 constitutes a regulating section which determines the direction of the observation unit 200 around the axis thereof.

As shown by a dotted line in FIG. 6, the receiving chamber 132 is formed into a shape which is laterally long, substantially flat, laterally symmetrical and vertically asymmetrical. That is, the cross-sectional shape of the receiving chamber 132 is adapted to that of the camera section unit 202. However, the cross-sectional shape of the receiving chamber 132 does not have to be a shape associated with the cross-sectional shape of the camera section unit 202, as long as the camera section unit 202 can be installed in the receiving chamber 132 while being positioned in a predetermined direction.

As shown in FIGS. 3A, 3B and 5, the rear end side inlet portion of the receiving chamber 132 is formed to spread vertically and horizontally and spread rearward in a substantially tapered shape as compared with the distal end side portion of the receiving chamber 132. The rear end side inlet portion of the receiving chamber 132 having the spreading shape forms an inlet guide portion (an insertion guide mechanism) 134 for inserting the camera section unit 202 into the receiving chamber 132. The inlet guide portion 134 has a guide function for smoothly guiding the portion of the camera section unit 202 guided by the guide tube 96 to a predetermined position in the receiving chamber 132.

The receiving chamber 132 is provided with a mechanism which positions and fixes the camera section unit 202 received in the receiving chamber 132. The distal end portion of the observation unit 200 can be positioned and fixed by the positioning/fixing mechanism. This is one example of the observation unit positioning/fixing mechanism. Moreover, as the camera section unit positioning/fixing mechanism, as shown in FIGS. 3A, 3B and 5, an edge portion 105 smaller than the inner space of the receiving chamber 132 is formed in the distal end edge of the observation opening 104. The edge portion 105 is a stopper which hits against the distal end peripheral edge of the camera section unit 202 received in the receiving chamber 132 to regulate the dead end position of the camera section unit 202 in an inserting direction. Moreover, a peripheral groove 139 into which an O-ring 140 as a seal member is fitted is formed in the inner surface of the observation opening 104 near the distal end of the observation opening. When the O-ring 140 is fitted into the peripheral groove 139 and the camera section unit 202 is received in the receiving chamber 132 as shown in FIG. 3B, the O-ring 140 serves as a liquid-tight mechanism which fastens the outer periphery of the distal end portion of the camera section unit 202 to seal the observation opening 104, and the O-ring further performs a positioning/holding function of fixing the camera section unit 202 positioned and arranged in the receiving chamber 132, at the predetermined position of the unit 202. Thus, the mechanism which positions and arranges the camera section unit 202 in the receiving chamber 132 and the mechanism which fixes the camera section unit 202 received in the receiving chamber 132 position and fix the observation unit 200 inserted in the insertion guide mechanism of the insertion section main body 13 with respect to the insertion section main body 13. In consequence, the mechanisms constitute one of the observation unit positioning/fixing mechanisms which position and fix the camera section unit 202 in the insertion section main body 13.

As shown in FIGS. 3A and 3B, in the portion of the second rigid portion (distal end portion) 42 corresponding to the inlet guide portion 134 of the receiving chamber 132, the distal end portion of the guide tube 96 for the camera section unit is arranged. Here, as shown in FIGS. 3A, 3B and 5, the distal end portion of the guide tube 96 is fixed to the member of the second rigid portion 42. Since the inlet guide portion 134 of the receiving chamber 132 is formed in such a tapered shape that the inlet guide portion spreads and becomes larger than the diameter of the distal end portion of the guide tube 96 as described above, a part of the distal end portion of the guide tube 96 may enter the inlet guide portion 134.

Moreover, the distal end portion of the guide tube 96 is not fixed to the member of the second rigid portion 42, and the guide tube 96 may freely be arranged so that the distal end portion of the guide tube can move forward/backward in the axial direction. The guide tube 96 is disposed in a position deviating from the central axis of the insertion section main body 13, and hence when the third bending portion 44 or the flexible portion 46 is bent, the guide tube 96 moves forward/backward in the axial direction. However, when the distal end of the guide tube 96 is not fixed to the second rigid portion 42 and is freely arranged with respect to the inlet 134 of the receiving chamber 132, the guide tube 96 itself moves in accordance with the deformation of the third bending portion 44 and the flexible portion 46, and the deformation of the third bending portion 44 and the flexible portion 46 is not disturbed. Moreover, when the distal end of the guide tube 96 is arranged in the inlet 134 of the receiving chamber 132, even the guide tube 96 moving forward/backward is prevented from coming off the inlet 134, and the camera section unit 202 guided through the guide tube 96 can securely be led into the receiving chamber 132.

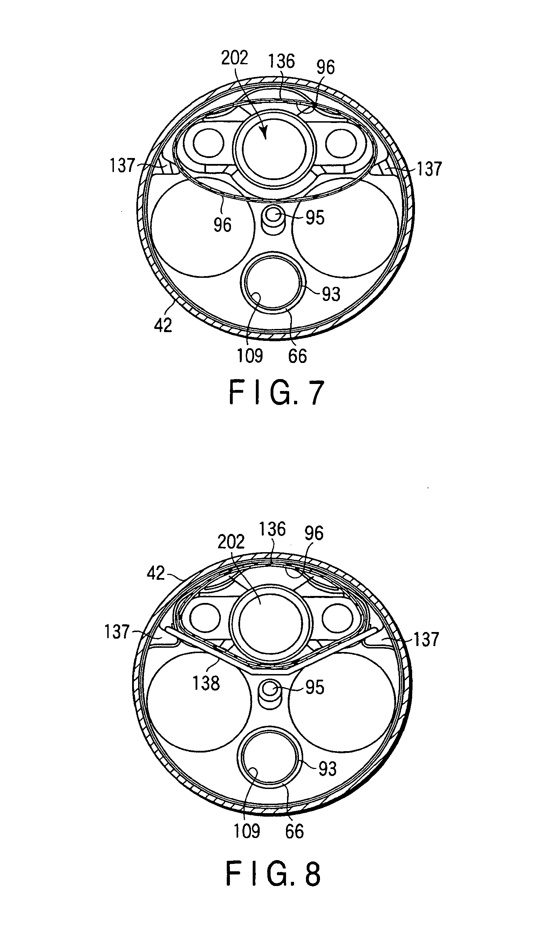

As shown in FIG. 7, a shape holding tape 136 is wound around the outer periphery of the distal end portion of the guide tube 96 to keep the shape of the distal end portion of the guide tube 96. Thus, when the shape holding tape 136 is wound around the outer periphery of the distal end portion of the guide tube 96, the shape of the distal end portion of the guide tube 96 is determined as a predetermined shape. In particular, even in a case where the distal end portion of the guide tube 96 is freely arranged so that a part of the distal end portion of the guide tube 96 enters the rear end side inlet portion, the position of the distal end of the guide tube 96 is easily determined. Moreover, as shown in FIG. 7, the distal end portion of the guide tube 96 is disposed on protrusions 137 provided on the inner surface of the second rigid portion 42 to install the guide tube 96 so that the distal end portion of the guide tube 96 is supported between the protrusions and the inner surface of the second rigid portion 42. In this case, the position of the distal end portion of the guide tube 96 is further easily determined. Moreover, the support portion of the distal end portion of the guide tube 96 may be bonded to the second rigid portion 42 and the protrusion 137. The guide tube positioning/fixing mechanism by the protrusion and the like is effective even for a case where any tape is not wound around the outer periphery of the distal end portion of the guide tube 96.

It is to be noted that the distal end of the guide tube 96 may be connected to the receiving chamber 132 through any other connection means for allowing the guide tube 96 to slide. Moreover, when the distal end of the guide tube 96 is fixedly connected to the receiving chamber 132 or the inlet 134, a stretchable elastic material may be used in the guide tube 96. Furthermore, expansion/contraction absorbing means may be constituted by incorporating an expanding/contracting or slidable member in a connecting portion between the guide tube 96 and the receiving chamber 132 or the inlet 134.

Furthermore, as shown in FIG. 8, both ends of a plate material 138 may be disposed on a pair of left and right protrusions 137 protruding from the inner surface of the second rigid portion 42, to support the distal end portion of the guide tube 96 from the downside by the plate material 138. The plate material 138 is formed of a thin metal plate or the like. The plate material 138 may be fixed to the protrusions 137 by bonding or the like. Moreover, the distal end portion of the guide tube 96 may be fixed to a peripheral portion by the bonding or the like. When the distal end portion of the guide tube 96 is fixed by the bonding or the like, the portion can securely be positioned and fixed.

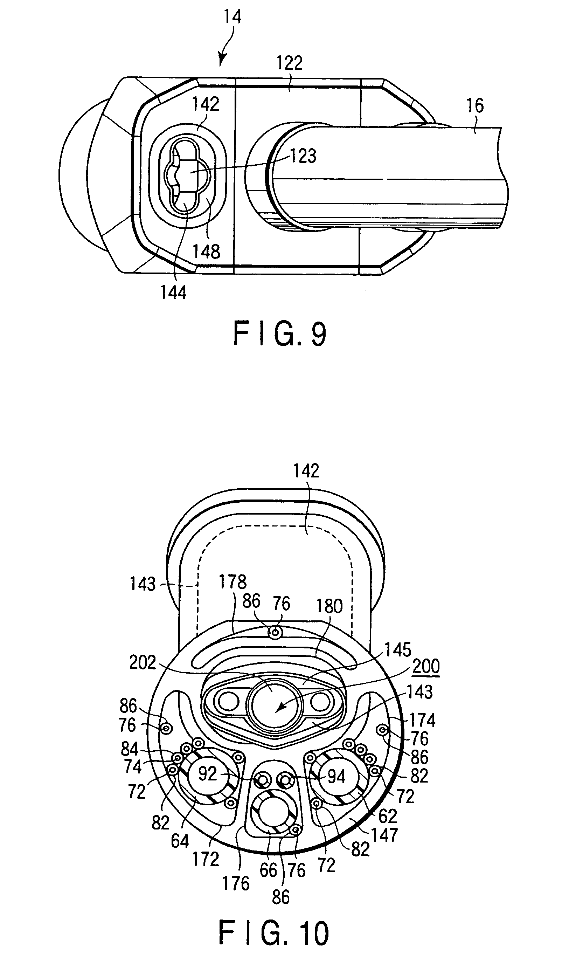

Next, a structure of the branching member 14 of the endoscope main body 100 will be described with reference to FIGS. 9 and 10. The branching member 14 is formed into a tripodal shape. Moreover, the branching member 14 branches the first extending section 16 and the second extending section 18 from the body cavity insertion section 12, and further branches the internal members arranged in the body cavity insertion section 12 into the first extending section 16 and the second extending section 18. Moreover, the branching member 14 has a function of holding the outwardly exposed rear end opening of the guide tube 96. The branching member 14 has a main body casing 122. The distal end portion of the main body casing 122 is connected to the proximal end portion of the body cavity insertion section 12. The first extending section 16 and the second extending section 18 are connected to the rear end portion of the main body casing 122.

As shown in FIG. 9, the rear end upper portion of the main body casing 122 is provided with an insertion port 123 as a port (a plug-in port) for inserting the observation unit (observation optical system) 200 into the insertion guide mechanism (guide tube 96). As shown in FIG. 2A, since the first extending section 16 is arranged along the same axis as that of the body cavity insertion section 12, the insertion port 123 is arranged so as to obliquely open outwardly in a direction opposite to the second extending section 18 with respect to the central axis of the first extending section 16. In consequence, when the observation unit 200 is inserted into the insertion port 123, the insertion is not disturbed by the first extending section 16 or the second extending section 18.

As shown in FIGS. 9 and 10, the guide member 142 formed into a cylindrical shape is incorporated in the main body casing 122 of the branching member 14. The guide member 142 constitutes the insertion guide mechanism which guides the observation unit 200 through a guide space 143 formed in the guide member 142, so that the observation unit 200 is regulated in predetermined upper, lower, left and right directions and inserted into the insertion path 97 of the guide tube 96 in the body cavity insertion section 12. The insertion port 123 is formed by an opening formed in one end of the guide member 142. The other end portion (an inner end portion) of the guide member 142 positions and holds the separation plate 147 described later, and is connected to the rear end opening edge of the guide tube 96.

The cross-sectional shape of the insertion port 123 is formed into a shape adapted to the cross-sectional outer shape of the camera section unit 202 of the observation unit 200. Here, the camera section unit 202 has a laterally symmetric and vertically asymmetric shape, and hence the cross-sectional shape of the insertion port 123 is adapted to the shape of the camera section unit 202. The insertion port 123 is formed into, for example, such a flat elliptic shape that the camera section unit 202 cannot rotate. Therefore, the insertion port 123 constitutes a rotation regulating section so that the camera section unit 202 cannot rotate in the guide member 142. Moreover, the insertion port constitutes a direction regulating section so that when the camera section unit 202 is inserted into the insertion port 123 and the upper, lower, left and right directions of the camera section unit 202 are not the predetermined directions, the camera section unit 202 cannot be inserted into the insertion port 123. Therefore, the guide member 142 including the insertion port 123 forms the direction regulating section which regulates the direction (posture) of the camera section unit 202 to be inserted. The portion of an outlet 145 of the guide space 143 shown in FIG. 10 may constitute a regulating section which regulates the direction (posture) of the camera section unit 202 in the same manner as in the insertion port 123.

Moreover, the portion of an inlet (an insertion guide section) 144 of the guide space 143 shown in FIG. 9 and the portion of the outlet (insertion guide section) 145 of the guide space 143 shown in FIG. 10 are formed into a cross-sectional shape closer to the outer shape of the camera section unit 202 as compared with another portion of the guide space 143. Thus, the shape of a small portion such as the inlet 144 or the outlet 145 is similar to the cross-sectional shape of the camera section unit 202, and is slightly larger than the camera section unit 202, whereby the guide function of the insertion guide section for regulating the inserting direction of the camera section unit 202 can be improved. The middle portion of the guide member 142 is comparatively thick, but has a similar flat shape. Therefore, the camera section unit 202 inserted from the inlet 144 of the guide member 142 is guided to the guide member 142 in specifically determined upper and lower directions, and the camera section unit keeping the posture thereof is guided to the guide tube 96 through the inlet 144 of the guide space 143 and the separation plate 147. Therefore, the camera section unit 202 inserted from the inlet 144 of the guide member 142 is brought closer to the center of the guide tube 96 of the insertion guide mechanism in the predetermined direction, and is guided to the guide tube 96.

On the other hand, as shown in FIG. 10, the separation plate 147 formed into a substantially disc-like shape is arranged at an inner end of the guide member 142 (distal end of the insertion section). The separation plate 147 includes first to fifth opening regions 172, 174, 176, 178 and 180 which are partitioned from one another. The first to third opening regions 172, 174 and 176 are arranged below the fifth opening region 180, and the fourth opening region 178 is arranged above the fifth opening region 180.

In the first opening region 172, the first channel tube 62 leading to the first arm section 32 is inserted, and the wires 72, 74 for bending the bending portions 38, 40 of the first arm section 32 and the wire guides 82, 84 covering these wires 72, 74 are inserted. In the second opening region 174, the second channel tube 64 leading to the second arm section 34 is inserted, and the wires 72, 74 for bending the bending portions 38, 40 of the second arm section 34 and the wire guides 82, 84 covering these wires 72, 74 are inserted. In the third opening region 176, the third channel tube 66, the air supply tube 92 and the water supply tube 94 are inserted. In the first to fourth opening regions 172, 174, 176 and 178, the third wires 76 for bending the third bending portion 44 and the third wire guides 86 covering these wires 76 are appropriately scattered and arranged. In the fifth opening region 180, the distal end portion of the above-mentioned guide member 142 and the proximal end portion of the above guide tube 96 are coaxially arranged, and the distal end portion of the guide member 142 is connected to partially enter the proximal end portion of the guide tube 96. In this case, the distal end portion of the guide member 142 and the proximal end portion of the guide tube 96 are fixed, but they may be connected to each other in a state in which they are slidably fitted while maintaining a communicating condition.

The separation plate 147 positions the distal end portion of the guide member 142 and the proximal end portion of the guide tube 96 at predetermined positions, and eventually any other internal member can separately be arranged away from the guide member 142 and the guide tube 96. Therefore, the guide tube 96 is arranged at a predetermined position solely independently from any other internal member. This determines the position of the guide tube 96 as the insertion guide mechanism for inserting the observation unit 200.

Here, as shown in FIG. 10, the first to third opening regions 172, 174 and 176 are arranged below the fifth opening region 180, and the fourth opening region 178 is arranged above the fifth opening region 180. Consequently, the first channel tube 62 leading to the first arm section 32, the pair of first wires 72, 74 for bending the bending portions 38, 40 of the first arm section 32, the first wire guides 82, 84 covering these wires 72, 74, the second channel tube 64 leading to the second arm section 34, the pair of second wires 72, 74 for bending the bending portions 38, 40 of the second arm section 34, the second wire guides 82, 84 covering these wires 72, 74, the third channel tube 66, the air supply tube 92, the water supply tube 94, the third wires 76, and the third wire guides 86 covering these wires 76 are arranged at separate positions while avoiding a region where the guide tube 96 as the insertion guide mechanism is arranged. Like the arrangement state of these members in the separation plate 147, the members are similarly arranged even in the second rigid portion 42 positioned at the distal end of the insertion section main body 13 in the same manner (see FIG. 11), and the distal end portions of the members arranged in this state are assembled to the second rigid portion 42.

Meanwhile, it is expected that the arrangement of each internal member incorporated into the insertion section main body 13 is always maintained in the above-mentioned predetermined positional relationship. However, since the third bending portion 44 and the flexible portion 46 of the insertion section main body 13 need to be allowed to curve or bend, the members incorporated in the insertion section main body 13 are basically arranged in a freely movable state in the insertion section main body 13. On the other hand, when the bending portion 44 and the flexible portion 46 curve or bend, the arrangement relationship of the members is apt to break down. To solve the problem, in the present embodiment, the insertion guide mechanism as the insertion path for guiding the observation unit 200 is also formed in the insertion section main body 13, and the observation unit 200 is guided to the distal end of the insertion section main body 13.

In the present embodiment, as shown in FIG. 11, to secure a passage region for inserting and guiding the observation unit 200 separately from the other internal members, the guide tube 96 as the insertion guide mechanism is partitioned and separated from the other internal members, and is arranged in the insertion section main body 13 of the body cavity insertion section 12. The insertion path (path) 97 for guiding the observation unit 200 by the guide tube 96 is secured in the insertion section main body 13. That is, the guide tube 96 forms the insertion path 97 for inserting and guiding the observation unit 200 separately from the internal members arranged in the flexible portion 46 and the third bending portion 44 of the insertion section main body 13. The distal end of the guide tube 96 is connected to the receiving chamber 132 of the second rigid portion 42.

Meanwhile, it is expected that the arrangement of the internal members incorporated in the insertion section main body 13 is always maintained in the above-mentioned predetermined positional relationship. However, since the third bending portion 44 and the flexible portion 46 of the insertion section main body 13 curve or bend, to allow the movement, the internal members are basically arranged in a free state in the insertion section main body 13. Therefore, when the third bending portion 44 and the flexible portion 46 curve or bend, the arrangement relationship of the internal members is apt to break down. When the observation unit 200 is simply inserted in the insertion section main body 13, the observation unit 200 cannot be inserted into the predetermined position.

To solve the problem, to define the insertion path for inserting and guiding the observation unit 200 separately from the other internal members, the guide tube 96 is arranged as a separation member in the insertion section main body 13 in a state where the guide tube 96 is partitioned and separated from the other internal members. Since the guide tube 96 is arranged in a partition separately from the other internal members, the guide tube 96 secures, in the insertion section main body 13, the insertion path (path) 97 which does not interfere with the other internal members and which guides the observation unit 200. Additionally, since the distal end of the guide tube 96 is connected to the receiving chamber 132 of the second rigid portion 42, the observation unit 200 can be guided to the receiving chamber 132. That is, the guide tube 96 is the separation member forming the insertion path 97 for inserting and guiding the observation unit 200, separately from the internal members arranged in the flexible portion 46 and the third bending portion 44 of the insertion section main body 13.

Meanwhile, as shown in FIGS. 1 and 2A, the observation unit 200 of the present embodiment includes the camera section unit 202 at the most distal end, the cable unit 204 connected to the camera section unit 202, and an observation unit connector 206 connected to the proximal end of the cable unit 204. The connector 206 for the observation unit is plugged in the connector 25 provided at the extending end of the universal cord 24, and is detachably attached. The connector 25 is provided with a signal cable 207. The extending distal end of the signal cable 207 is provided with a video connector 208 shown in FIG. 1. Moreover, the video connector 208 is connectable to a camera control unit as an external device (not shown). The camera control unit captures video imagery by a camera module 226 as a camera section of the observation unit 200, and converts, into a video signal, video signal data transmitted via a signal line (a signal transmission portion) 228 connected to the camera module 226, to display the imagery on a monitor (not shown). The connector 25 is also used as a light guide connector for connecting a light guide tube 203 to a light source device (not shown). Furthermore, the connector 25 is connected to a fluid source of a fluid control unit (not shown) through a connection terminal (not shown), to supply or extract air, water or the like to or from the air supply tube 92, the water supply tube 94 or the third channel tube 66 of the operative endoscope 10 through the above-mentioned universal cord 24. The light guide tube 203 is connected to a later-described light guide (a fiber bundle) 224 of the cable unit 204 for inducing illuminative light.

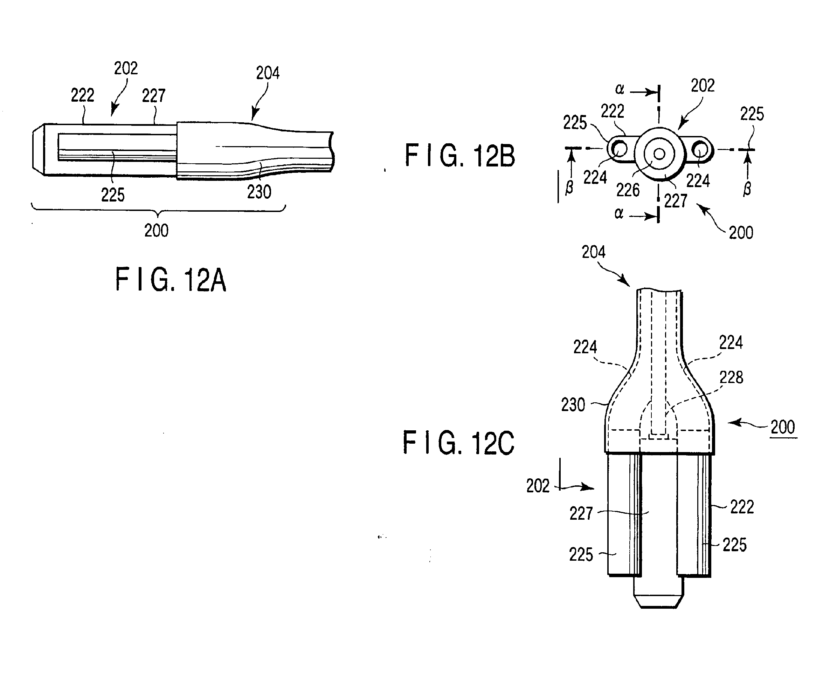

As shown in FIGS. 12A to 13A, the camera section unit 202 includes a casing 222 made of a metal material. As shown in FIG. 12B, in the casing 222 are integrally formed a pair of pipe sections 225 into which the distal end portions of the light guides 224 are inserted, respectively, and one pipe section 227 which is positioned between the pair of pipe sections 225 and into which the camera module (including an imaging device such as a CCD and an objective optical system) 226 for capturing imagery to be observed is inserted, so that these pipe sections 225, 227 are arranged in parallel with their longitudinal directions being set to the same direction. Moreover, as shown in FIG. 12B, the positions of the pair of left and right pipe sections 225 slightly deviate upwards from the center of the pipe section 227 placed at the center. Therefore, the pair of pipe sections 225 and the one pipe section 227 substantially have a laterally symmetrical shape with respect to line α-α as a vertical direction in FIG. 12B. Since the pipe sections 225 are arranged in the vertical direction with respect to the pipe section 227, that is, the sections 225 are offset upwards herein, these pipe sections 225 have an asymmetrical shape with respect to line β-β as a lateral direction. The casing 222 has an asymmetrical cross-sectional shape in at least one of the vertical direction and the lateral direction. Moreover, the cross-sectional shape of the camera section unit 202 is an irregular shape which does not have any symmetric properties in at least one of the vertical direction and the lateral direction. Furthermore, it is a flat cross-sectional shape in which a vertical width is different from a lateral width. In this example, it is a flat shape in which the vertical width is smaller than the lateral width.

Although the cross-sectional shape of the casing 222 is laterally symmetrical with respect to line α-α alone in the above explanation of this embodiment, it may be an asymmetrical shape even with respect to line α-α. In this case, it becomes an irregular shape which does not have any symmetric properties in both the vertical direction and the lateral direction.

Therefore, the upper, lower, left and right directions of the camera section unit 202 are specified. In consequence, the camera section unit 202 having the specified directions is inserted into the insertion path 97 of the insertion guide mechanism of the operative endoscope 10 and disposed in the receiving chamber 132 in the specified directions. As described above, both the insertion path 97 and the receiving chamber 132 have such flat cross-sectional shapes that the camera section unit 202 cannot be inserted/disposed unless the specified directions are used. As described above, the insertion port 123 of the branching member 14 and the guide space 143 in the guide member 142 likewise have flat cross-sectional shapes. Therefore, the camera section unit 202 is not reversed while the unit is inserted into the insertion guide mechanism formed between the insertion port 123 of the branching member 14 and the separation plate 147 and between the separation plate 147 and the receiving chamber 132, and the unit is guided in the predetermined posture, and attached to the body cavity insertion section 12. In particular, the insertion port 123 and the outlet 145 of the guide member 142 are narrowly formed in accordance with the irregular cross-sectional shape of the camera section unit 202 which uniquely determines the vertical and lateral directions. In consequence, the camera section unit 202 in a vertically inverse direction cannot be inserted into the insertion port 123.

As shown in FIGS. 12A to 13B, the signal line (signal transmission portion) 228 and the light guides (illumination portions) 224 which are connected to the camera module 226 are bound up into one cable by a binding member such as a thermally shrinkable tube 230, thereby constituting the cable unit 204. The distal end portion of the thermally shrinkable tube 230 covers the proximal end portion of the casing 222, whereby the thermally shrinkable tube 230 functions as an anti-folding tube with respect to the casing 222. The cable unit 204 has flexibility, but serves as an introduction lead section when inserting or removing the camera section unit 202 into or from the insertion guide mechanism, so that the cable unit needs to have such high elasticity that an introducing operation force thereof can be transmitted.

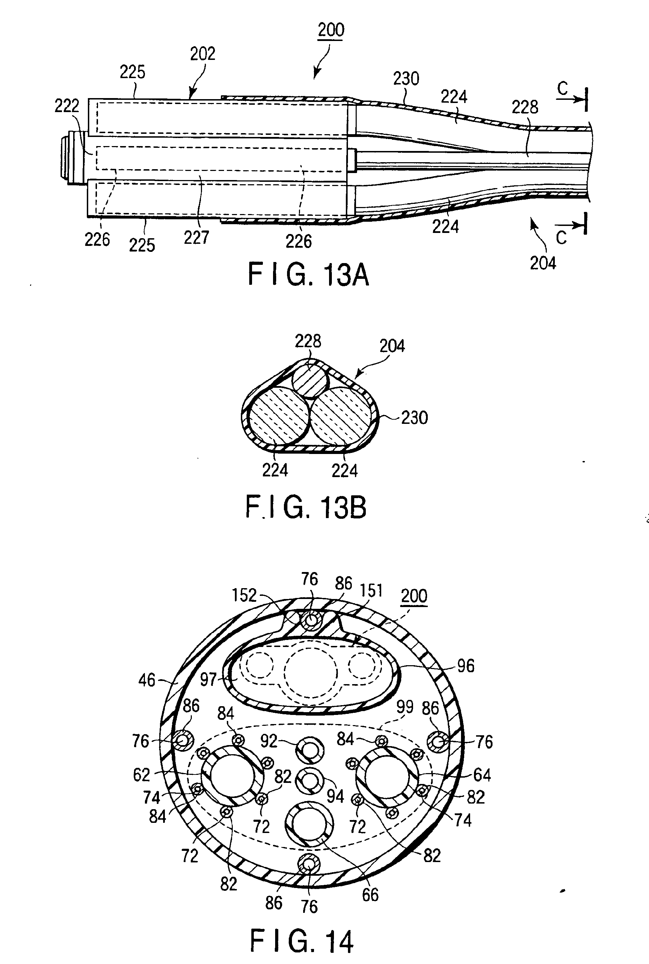

In this example, as shown in FIGS. 13A and 13B, since the signal line 228 and the pair of light guides (fiber bundles) 224 are integrally bound up by a binding member such as the thermally shrinkable tube 230, predetermined elasticity is assured.

Moreover, as shown in FIGS. 1 and 2A, the middle of a cable portion of the cable unit 204 is provided with a cable operating grasp portion 205. As shown in FIGS. 1 and 2A, an engaging portion 209 which also serves as an anti-folding member is provided between the cable operating grasp portion 205 and the distal end cable portion. The engaging portion 209 can disengageably be engaged with an engagement portion provided in an opening portion 148 of the inlet 144 of the branching member 14. Here, a seal portion may be provided to liquid-tightly seal a part between the engaging portion 209 and the engagement portion provided in the opening portion 148 of the inlet 144 of the branching member 14 in an engaged state.

The cable operating grasp portion 205 is a pushing operation member for grasping the cable unit 204 when pushing the cable unit 204 into the insertion section main body 13 of the operative endoscope 10. Therefore, when the observation unit 200 is attached to the insertion guide mechanism of the body cavity insertion section 12, the cable operating grasp portion 205 is grasped to push the observation unit 200 inwards, and moreover a pushing amount can be adjusted. Moreover, the engaging portion 209 can be fitted into and engaged with the insertion port 123 of the insertion guide mechanism provided in the branching member 14, to fixedly position the cable unit 204 with respect to the body cavity insertion section 12.

When the cable unit 204 is inserted into the body cavity insertion section 12 from the insertion port 123 and the observation unit 200 is mounted on the operative endoscope 10, the portion of the cable unit 204 which starts to be led out of the operative endoscope 10 is covered with the cable operating grasp portion 205, so that the lead-out proximal end portion of the cable unit 204 is prevented from being suddenly folded.

Moreover, the length of the cable unit 204 is set so that the camera section unit 202 of the observation unit 200 is received in the receiving chamber 132 of the body cavity insertion section 12, when the engaging portion 209 at the distal end of the cable operating grasp portion 205 is engaged with the branching member 14 of the body cavity insertion section 12. Therefore, when the observation unit 200 is attached to the insertion guide mechanism of the body cavity insertion section 12 and the engaging portion 209 is engaged with the branching member 14, the observation unit 200 is positioned at the predetermined position with respect to the body cavity insertion section 12, and fixed in this state. Therefore, the engagement portion with respect to the branching member 14 is also one example of the observation unit positioning/fixing mechanism for positioning and fixing the observation unit 200 with respect to the body cavity insertion section 12.

Next, a function when using the operative endoscope 10 according to this embodiment will be described. When using the operative endoscope 10, the observation unit 200 is inserted into the insertion port 123 of the endoscope main body 100. At this time, if the camera section unit 202 of the observation unit 200 is not inserted in the predetermined upper, lower, left and right directions, the camera section unit cannot be inserted into the insertion port 123. Therefore, it can be judged whether or not the observation unit 200 has a correct direction by judging whether or not the camera section unit 202 can be inserted into the insertion port 123, and the observation unit 200 can be inserted in the correct direction. Moreover, the observation unit 200 in the predetermined upper, lower, left and right directions is inserted into the guide tube 96 from the insertion port 123 by the insertion guide mechanism, and the camera section unit 202 is guided to the receiving chamber 132.

The guide tube 96 is separated from the other internal members and arranged in the insertion section main body 13 to secure the insertion path 97 of the insertion guide mechanism. In consequence, the camera section unit 202 of the observation unit 200 can be smoothly guided to the receiving chamber 132 through the insertion path 97 without interfering with the other internal members. Moreover, the insertion path 97 is formed into a cross-sectional shape associated with the outer shape of the camera section unit 202. Consequently, the camera section unit 202 can be led to the receiving chamber 132 in the predetermined direction, and inserted and received in the receiving chamber 132 while keeping the direction. Moreover, the camera section unit 202 attached to the insertion section main body 13 is positioned and fixed by the observation unit positioning/fixing mechanism (edge portion 105 of the receiving chamber 132 or the O-ring 140). The distal end of the camera module 226 in a predetermined direction is fixed to a predetermined position with respect to the observation opening 104. Moreover, the distal end of each light guide 224 is positioned in the illumination window 102. The cable operating grasp portion 205 can be engaged, positioned and fixed with respect to the branching member 14 of the operative endoscope 10 to position and fix the observation unit 200 at a predetermined attachment position with respect to the operative endoscope 10. In consequence, the attachment of the observation unit 200 is completed. The operative endoscope 10 has an illumination function and an observing function by the observation unit 200, and is ready for use. The portion of the observation unit 200 attached to the insertion section main body 13 and inserted into the body cavity insertion section 12 is attached to the body cavity insertion section 12 from the outside in a liquid-tight state.

Next, a case where the assembled operative endoscope 10 is used will be described. First, the body cavity insertion section 12 is inserted into the body cavity. While observing the inside of the body cavity by the operative endoscope 10, the third bending portion 44 is operated and bent by the first operating section 20, and the first arm section 32 and the second arm section 34 can be bent by the second operating section 22. Moreover, when a surgical instrument is inserted into the body cavity through the channels 62, 64 and 66 provided in the operative endoscope 10 to carry out a procedure or the like, a multifunctional procedure can be performed. Moreover, a procedure such as liquid supply/suction can be performed through the channels 62, 64 and 66. According to the operative endoscope 10, the multifunctional procedure can be performed with low invasiveness.

After the use of the operative endoscope 10, the observation unit 200 is removed from the endoscope main body 100, and the observation unit 200 may be cleaned, disinfected, sterilized and then reused. Moreover, the endoscope main body 100 may be cleaned, disinfected, sterilized and reused, or may be discarded.

That is, since the endoscope main body 100 according to this embodiment is provided with the observation unit attachment/detachment mechanism capable of removing or inserting the observation unit 200, the observation unit 200 can be detachably attached to the body cavity insertion section 12 on the side of the endoscope main body 100. When the observation unit 200 is attached to the endoscope main body 100, a burden imposed on an operator is decreased. Moreover, the body cavity insertion section 12 on the side of the endoscope main body 100 including the operating arm sections 32, 34 can be separated from the observation unit 200. Therefore, for example, in a case where the endoscope 10 is used, the body cavity insertion section 12 of the endoscope main body 100 is discarded, the observation unit 200 is cleaned, and the endoscope main body 100 only may be replaced with a new endoscope main body 100. Consequently, cost required for a cleaning operation can noticeably be decreased. When the expensive observation unit 200 including a large number of electronic components, for example, an imaging device is reused, the cost per operation can be decreased.

Although the case where the pair of operating arm sections 32, 34 are arranged in the endoscope main body 100 has been described in this embodiment, three or more operating arm sections may be provided. Furthermore, one operating arm section may be provided.

### [Second Embodiment]

Next, a second embodiment according to the invention will be described.

FIG. 14 shows a modification of the guide tube 96 in the above-mentioned operative endoscope 10. An upwardly protruding portion 151 is provided on an upper portion of the guide tube 96 shown in FIG. 14. The protruding portion 151 is provided with an engagement concave portion (a positioning portion) 152 which is engaged with a third wire guide 86 positioned on the upside as shown in FIG. 14. In this embodiment, the protruding portion 151 may be continuously formed over the entire length of the guide tube 96, but it is better to partially arrange the protruding portions at intervals in the longitudinal direction of the guide tube without being continuously formed in order to improve the flexibility of an insertion section 12.

Since this embodiment is provided with the positioning portion for engaging a third wire guide 86 with the engagement concave portion 152 to regulate the position of the third wire guide 86 in a flexible portion 46, the position of the guide tube 96 in the flexible portion 46 can be determined, thereby stabilizing the position of an insertion path 97. Furthermore, since the guide tube 96 is received by a group of other internal members 99 from the lower side of the guide tube, the guide tube 96 is positioned in vertical and lateral directions in the flexible portion 46, thus stabilizing the position and the posture of the insertion path 97 of an insertion guide mechanism.

According to this embodiment, the guide tube 96 having the insertion path 97 through which an observation unit 200 can be removed from/inserted into an insertion section main body 13 is secured separately from the group of the other internal members 99, and the position of the guide tube 96 can be stabilized. Therefore, the observation unit 200 can smoothly be led to a predetermined portion near the distal end of the insertion section main body 13 through the insertion path 97 formed by the guide tube 96.

### [Third Embodiment]

Next, a third embodiment will be described.

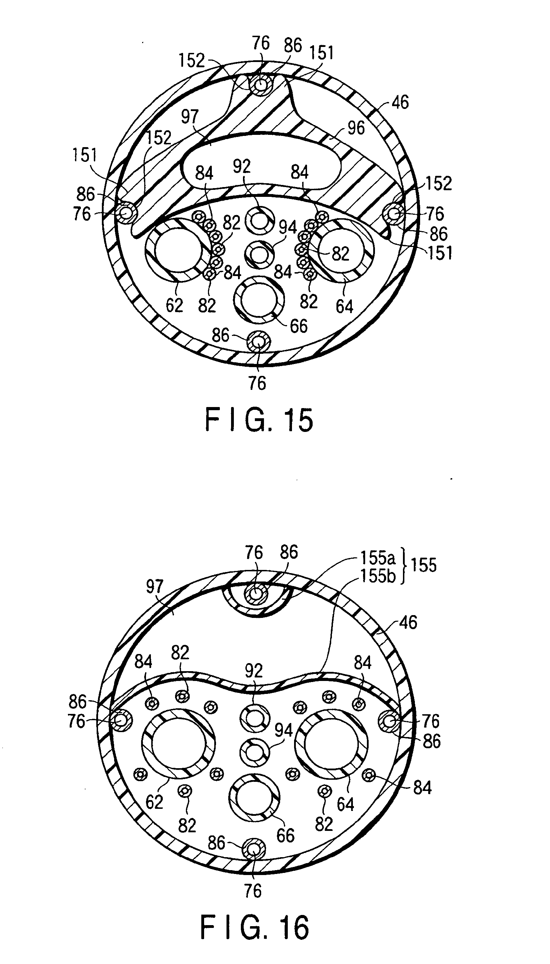

A configuration shown in FIG. 15 is a modification of the second embodiment shown in FIG. 14 (a first modification of the second embodiment). In this embodiment, protruding portions 151 and engagement concave portions 152 (positioning portions) are formed on not only the upside of FIG. 15 but also left and right side surface portions of the guide tube 96, and the engagement concave portions 152 are engaged with third wire guides 86 to determine the positions of the portions. Therefore, the guide tube 96 is supported by the third wire guides 86 in the respective left, right and upper directions, thus increasing the stability of the position. Furthermore, since the guide tube 96 is received by the other internal members from the downside, the guide tube 96 is positioned in all of the vertical and lateral directions in a flexible portion 46, and hence the stability of the position and posture of an insertion path 97 is improved.

### [First Example helpful for understanding the invention]

Next, a first example will be described.

FIG. 16 shows another modification of the insertion path 97 in the above operative endoscope 10. In this modification, any guide tube 96 is not used, and a wall portion (a guide portion) which guides an observation unit 200 into a third bending portion 44 and a flexible portion 46 forms the insertion path 97. That is, walls (partition walls) 155 (155a, 155b) which achieve separation from internal members other than the observation unit 200 are provided to form the insertion path 97 constituted of the wall portion (guide portion) for guiding the observation unit 200 between the walls. As the walls 155, there are used a first wall 155a positioned on the upside to surround a third wire guide 86 and a second wall 155b positioned on the downside and arranged to cover a group of the other internal members. A separation member which forms the insertion path 97 into which the observation unit 200 is inserted is provided between the first wall 155a and the second wall 155b. The separation wall (separation member) 155 forms the insertion path 97 in the third bending portion 44 and the flexible portion 46. Therefore, the observation unit 200 can be guided by the insertion path 97 formed between the first wall 155a and the second wall 155b. Moreover, the shape of the insertion path 97 is defined so that a camera section unit 202 is guided in a predetermined direction without being rotated in the insertion path 97. Even in this case, the passage shape of the insertion path 97 is determined so that the camera section unit 202 is guided in a predetermined direction without being rotated in the insertion path 97.

Meanwhile, the walls 155 may be continuously formed in the longitudinal axis direction of an insertion section main body 13, but the walls may partially be arranged at intervals in the longitudinal direction of the insertion section main body 13 without being continuously formed in order to improve the flexibility of the insertion section main body 13. Each of the first wall 155a and the second wall 155b may be formed into a sheet-like shape by using a resin sheet or a metal foil so that the walls 155 can have flexibility enabling deformation thereof in accordance with the deformation of the insertion section main body 13.

### [Second Example helpful for understanding the invention]

Next, a second example will be described.

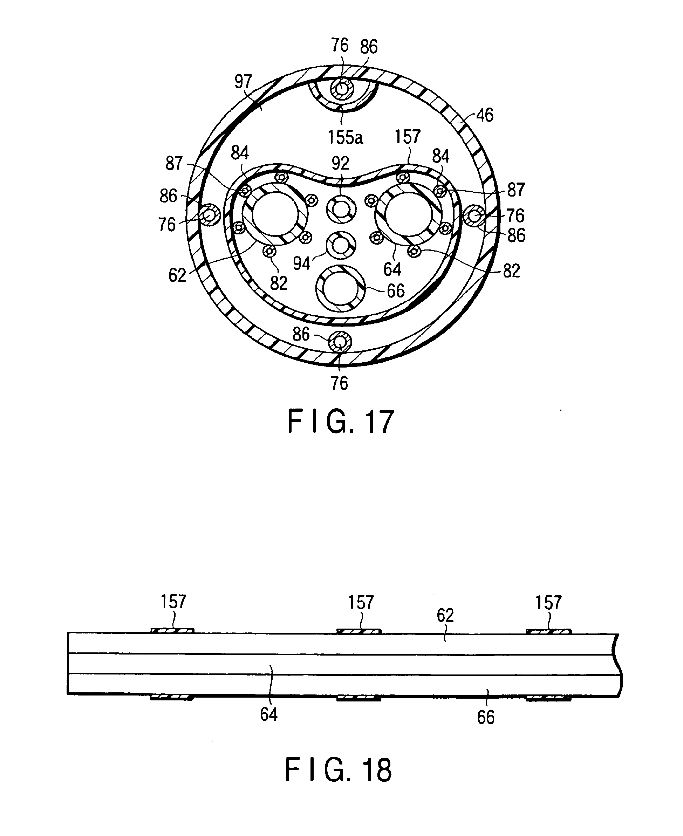

FIGS. 17 and 18 show a modification of the above separation member (wall). As to a separation member in this example, a tube member 157 is used as a binding member for binding internal members other than third wire guides 86 in a third bending portion 44 and a flexible portion 46. When the internal members other than the third wire guides 86 are bound by the tube member 157, an insertion path 97 in which an observation unit 200 is inserted is formed and partitioned from the internal members. The separation member is the tube member 157 which surrounds and bundles the internal member as shown in FIGS. 17 and 18. Since the binding member separately forms the insertion path 97 for inserting the observation unit 200 separately from the internal members arranged in the flexible portion 46 and the third bending portion 44 of an insertion section main body 13, the insertion path 97 into which the observation unit 200 is inserted can easily be secured. In this embodiment, as the member forming the insertion path 97, a partition wall 155a which forms the insertion path separately from the internal members other than the observation unit 200 may be used.

The tube member 157 may be a thermally shrinkable tube or tape or the like, and for example, the tube members bind the internal members at several positions as shown in FIG. 18. The tube member 157 as the binding member serves as a wall (partition wall) which forms the insertion path 97 partitioned from the internal members other than the observation unit 200. The tube member 157 may continuously be formed, but as shown in FIG. 18, tube members may partially be arranged at intervals without being continuously formed, to improve the flexibility of an insertion section.

### [Third Example helpful for understanding the invention]

Next, a third example will be described.

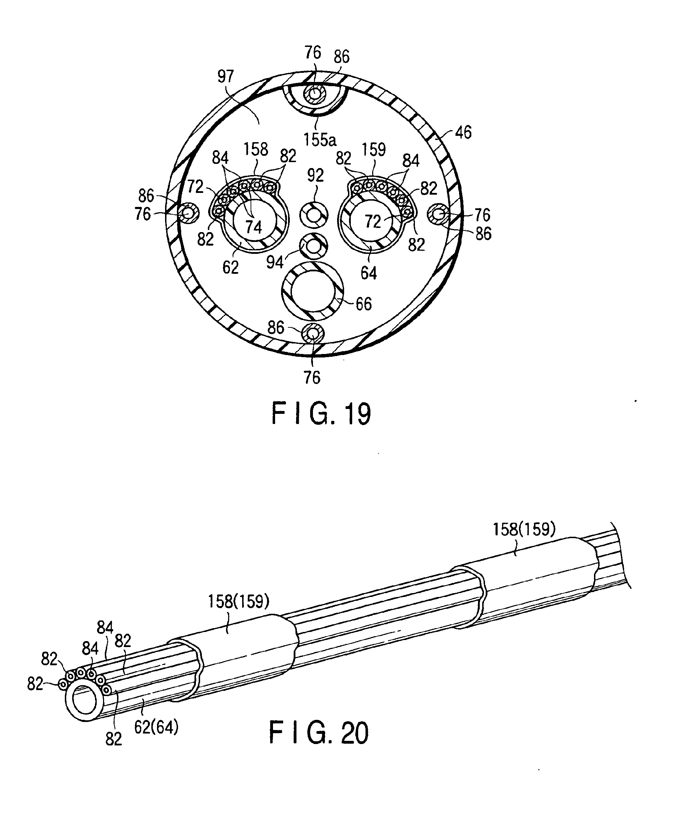

FIGS. 19 and 20 show another modification of the separation member (wall). In this example, (first) wire guides 82 of first bending portions 38 and (second) wire guides 84 of second bending portions 40 are bundled by a binding member (a first binding member) 158 while the wire guides come in contact with the outer periphery of a first channel tube 62. Moreover, the first wire guides 82 and the second wire guides 84 are also bundled by a binding member (a second binding member) 159 while the wire guides come in contact with the outer periphery of a second channel tube 64. When the sets of the respective channel tubes 62, 64 and the associated wire guides 82, 84 are bundled by the respective binding members 158, 159, the respective channel tubes 62, 64 and the respective wire guides 82, 84 are collected up without being scattered, and portions near the tubes are not complicated. Furthermore, the first binding member 158 and the second binding member 159 serve as a guide portion forming one wall portion of an insertion path 97, the insertion path 97 can separately be formed above these binding members, and a space for the insertion path can stably and continuously be formed. In this embodiment, the first binding member 158 and the second binding member 159 are separation members which form the insertion path 97 separately from the other internal members.

It is to be noted that each binding member 158 or 159 may be a thermally shrinkable tube or tape or the like. Moreover, the internal members may be bundled at several positions as shown in FIG. 20.

### [Fourth Example helpful for understanding the invention]

Next, a fourth example will be described.

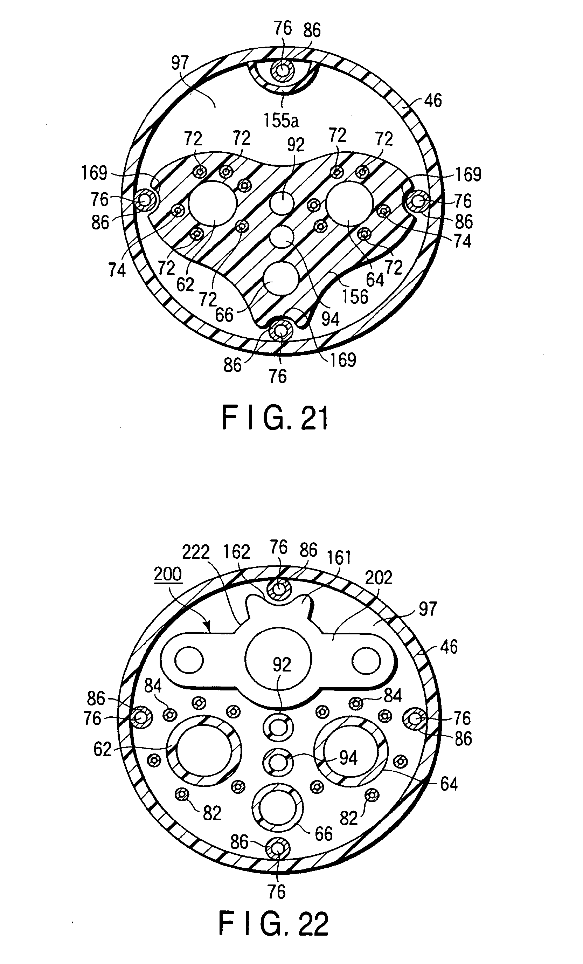

The example shown in FIG. 21 is a modification (a modification of the third example) of the separation member (wall). In particular, internal members other than third wire guides 86 are incorporated into a multi-lumen tube 156. The multi-lumen tube 156 is a separation member (separation means) which leaves a space remaining above the multi-lumen tube 156 as an insertion path 97 as a guide portion. Engagement concave portions 169 (positioning portions) which engage with the corresponding left, right, and lower third wire guides 86 are provided on the left and right side surfaces and the lower surface of the multi-lumen tube 156, respectively. These engagement concave portions 169 can stabilize the position of the multi-lumen tube 156 in an insertion section main body 13. In this embodiment, since a plurality of internal members are arranged by utilizing lumens of the multi-lumen tube 156, members for the respective internal members can be omitted or the plurality of internal members can compactly be collected. Moreover, since the arbitrary movement of the internal members can be suppressed, the shape of the insertion path 97 can be stabilized, and the insertion performance of an observation unit 200 improves.

### [Fourth Embodiment]#

Next, a fourth embodiment will be described.

The embodiment shown in FIG. 22 shows a modification of the camera section unit 202 of the observation unit 200 and the insertion path 97. A basic structure of the camera section unit 202 in this embodiment is described above, but an upwardly protruding portion 161 in FIG. 22 is formed on the upper surface portion of a casing 222 at the distal end of the camera section unit 202. An engagement concave portion 162 (a positioning portion) which engages with a third wire guide 86 positioned on the upside is formed in the protruding portion 161.

Moreover, when inserting the observation unit 200 into the insertion path 97, the engagement concave portion 162 of the camera section unit 202 is led along the third wire guide 86 positioned on the upside to guide the camera section unit 202 by using the engagement concave portion 162 as a guide portion. When such a guide portion is provided in the camera section unit 202, the camera section unit 202 can smoothly be inserted without wobbling in the insertion path 97 disposed in an insertion section main body 13, and the camera section unit 202 can smoothly be inserted or removed.

### [Fifth Embodiment]

Next, a fifth embodiment will be described.

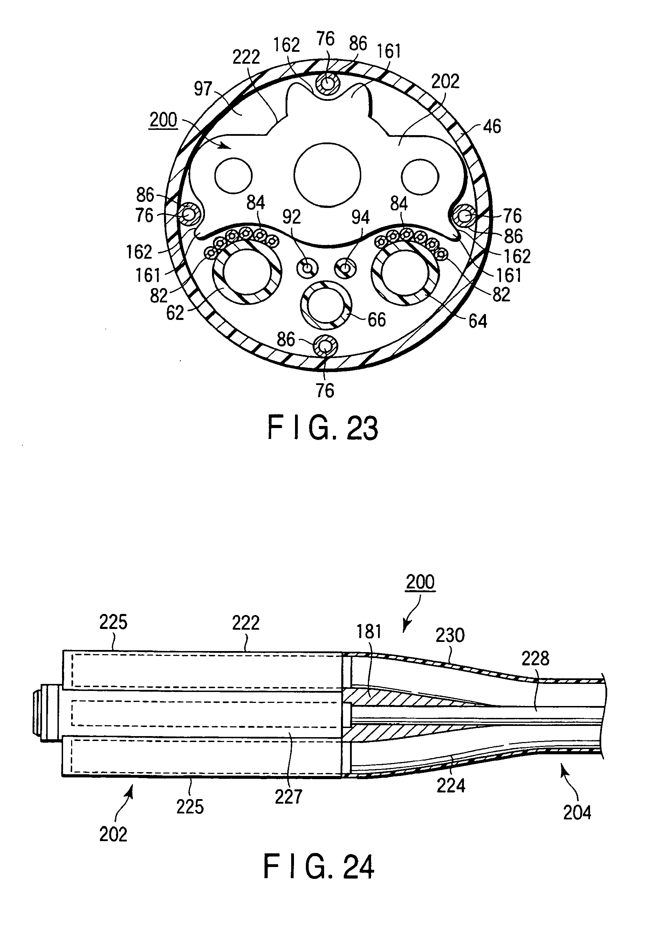

FIG. 23 shows a modification of the camera section unit 202 of the observation unit 200 and the insertion path 97. In this embodiment, engagement concave portions (positioning portions) 162 are formed not only on the upside of the camera section unit 202 shown in FIG. 23 but also on the left and right side surfaces of the camera section unit 202, and third wire guides 86 positioned on the left and right sides are engaged with the left and right engagement concave portions 162. Since the engagement concave portions 162 are formed as guide portions on the upside and the left and right side surfaces of the camera section unit 202, the wobbling motion of the camera section unit 202 can further be suppressed in the insertion path 97, and the camera section unit 202 can smoothly be inserted into/removed from the insertion path 97.

### [Sixth Embodiment]

Next, a sixth embodiment will be described.

FIG. 24 shows a modification of the camera section unit 202 of the observation unit 200 and the insertion path 97. In the observation unit 200, the proximal end portion of a casing 222 of the camera section unit 202 is not covered with a binding member such as a thermally shrinkable tube 230, and is led out from the rear end of the casing 222 of the camera section unit 202. In particular, a signal line 228 and a pair of light guides 224 are covered with the binding member 230, that is, the thermally shrinkable tube or the like, and the distal end of the binding member 230 is held at the rear end of the casing 222 so that it does not protrude from the outer periphery of the casing (is not covered). The led-out portions of the members (228, 224) led out from the casing 222 are fixed by a filler 181 filled in the binding member 230, and are provided with an anti-folding function. It is to be noted that the filler 181 may be a known material such as an adhesive, a sealing agent or a resin, and there is not any special restriction on the filler.

### [Seventh Embodiment]

Next, an seventh embodiment will be described.

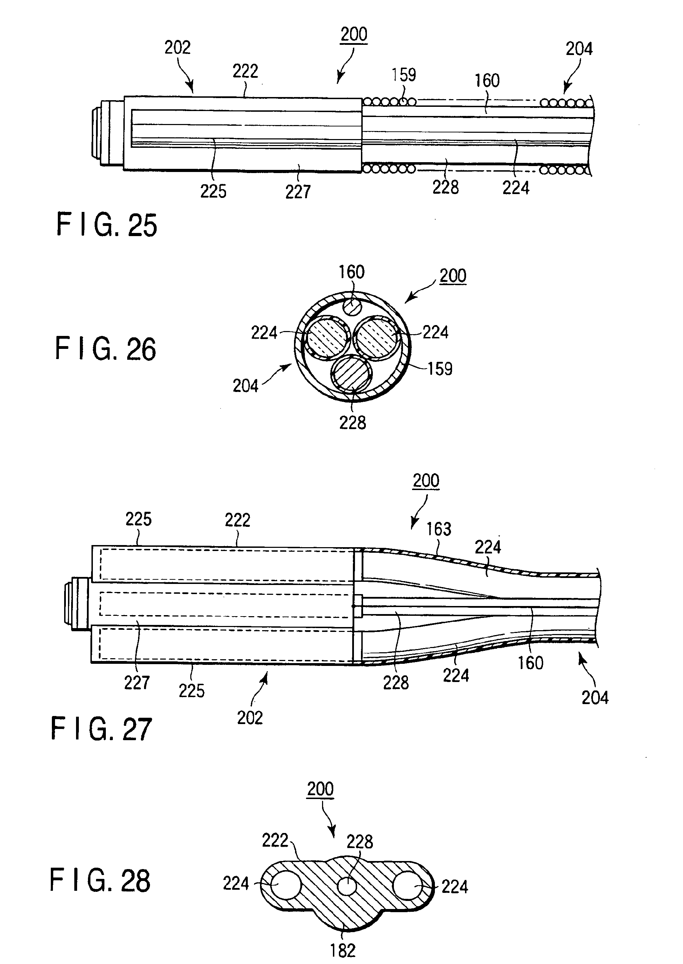

The embodiment shown in FIGS. 25 and 26 shows a modification of the observation unit 200 of each above embodiment. In this embodiment, members of a cable unit 204 are covered with coils 159. The distal end of the coil 159 is connected to the rear end of a casing 222 by soldering or the like. A draw-wire (a rigid linear member) 160 is connected to the rear end of the casing 222. The draw-wire 160 is led to the operator's hand side of the cable unit 204 through the coil 159.

Furthermore, when inserting the observation unit 200 into an operative endoscope 10, the observation unit is pushed inwards by using the coil 159. When removing the observation unit 200 from an endoscope main body 100 of an operative endoscope 10, the draw-wire 160 is pulled to remove the observation unit 200.

Even if the coil 159 is not fixed to the casing 222, a function of pushing the observation unit 200 inwards can be obtained, and hence the coil can be used without being fixed to the casing 222.

### [Eight Embodiment]

Next, an eighth embodiment will be described.

As shown in FIG. 27, a pipe 163 may be used instead of the coil 159. The pipe 163 slidably covers a signal line 228 and a pair of light guides 224 as members of a cable unit 204.

Moreover, when an observation unit 200 is mounted on an endoscope main body 100, the distal end of the pipe 163 is attached to the rear end of a casing 222 to push the whole observation unit 200 inwards.

On the other hand, after mounting the observation unit 200 on the endoscope main body 100 of an operative endoscope 10, the pipe 163 is extracted. To remove the observation unit 200 from the endoscope main body 100 of the operative endoscope 10, the draw-wire 160 is utilized to pull out the observation unit 200. The distal end portion of the pipe 163 is formed into such a tapered shape that the distal end side thereof spreads. It is to be noted that the draw-wire 160 can be utilized even in a configuration of the observation unit 200 which does not use any coil or pipe.

### [Ninth Embodiment]

Next, a ninth embodiment will be described.

In the embodiment shown in FIG. 28, cable members including a signal line 228, a pair of light guides 224 and the like in a cable unit 204 are integrated by extrusion forming.

In this embodiment, the cable members are bound by a resin 182, integrated, and readily collected as a whole. Moreover, the cross-sectional shape of the cable member (cable unit 204) can be easily selected. When the cable unit 204 is formed to have a cross-sectional shape similar to that of the camera section unit 202 shown in FIG. 27, the insertion guiding performance of the whole observation unit 200 can be improved.

### [Tenth Embodiment]

Next, a tenth embodiment will be described.

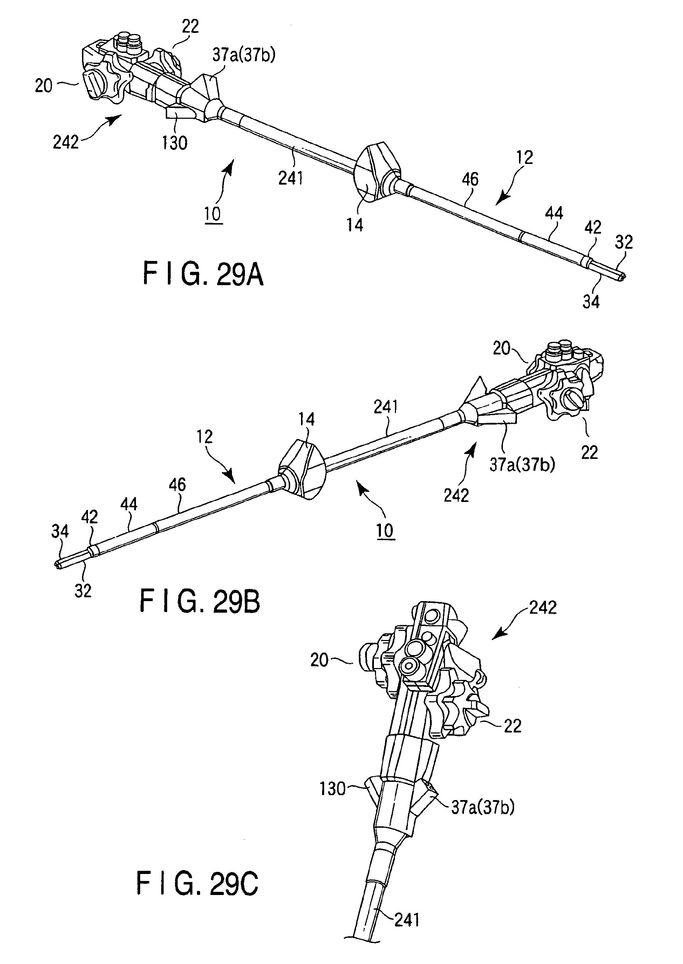

The embodiment shown in FIGS. 29A to 29C shows another modification of the endoscope main body 100 of the operative endoscope 10. In this modification, one flexible extending portion 241 extends from the proximal end of a body cavity insertion section, and the extending distal end of the extending portion 241 is provided with an operating section 242. The operating section 242 includes an operation mechanism having the functions of a first operating section (a main body operating section) 20 and a second operating section (an arm section operating section) 22. Therefore, the first operating section 20 is not disposed away from the second operating section 22, and the sections are disposed close to each other, so that one operator easily operates both the operating sections 20, 22. An integral system in which the first and second operating sections 20, 22 are collected at one place as in such the operating section 242 is suitable for a case where the operator alone uses the operative endoscope 10 without any assistance. The other constitution and the like may be similar to those described above.

### [Eleventh Embodiment]

Next, an eleventh embodiment will be described.

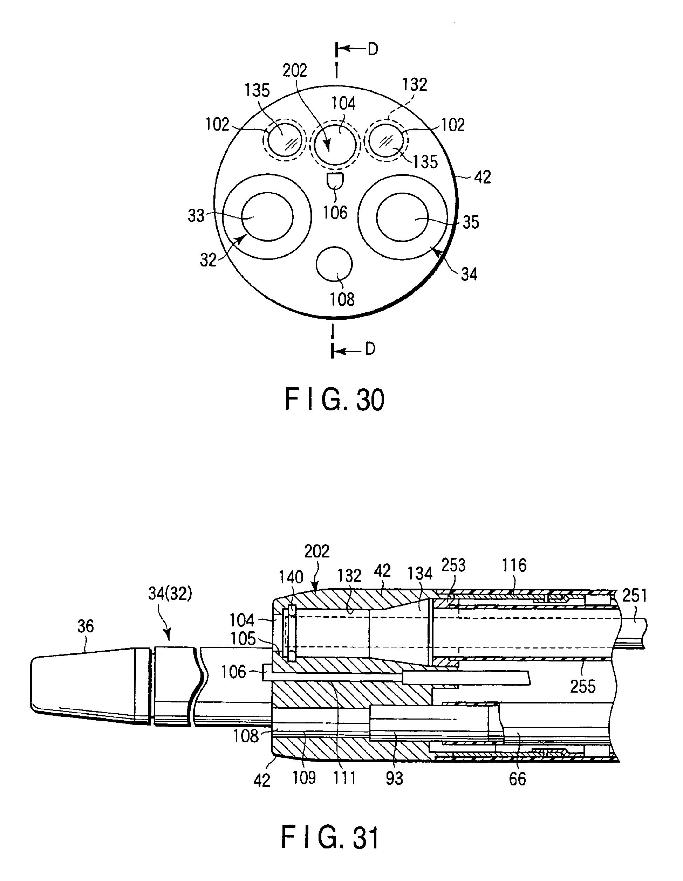

FIGS. 30 to 35 show still another modification of the endoscope main body 100 of the operative endoscope 10. In this embodiment, an observation unit 200 attachable to/detachable from a body cavity insertion section 12 is constituted of an only camera section unit 202 which does not include any illumination system. An illumination mechanism is constituted of a light guide 251 including a fiber bundle and the like separately from the observation unit 200, and is incorporated in the endoscope main body 100 separately from the observation unit 200.

As shown in FIG. 31, a holding member 253 for holding the distal end of the light guide 251 is fixedly provided at a second rigid portion (a distal end portion) 42 positioned at the most distal end of an insertion section main body 13, and the holding member 253 is a positioning portion which determines the position of the distal end of the light guide 251 in the second rigid portion 42.

Moreover, the distal end of the light guide 251 is positioned and fixed to the inner end position of each illumination window 102 shown in FIG. 30. The proximal end portion of the light guide 251 is led from a universal cord 24 to a connector 25 through a first extending section 16 and a first operating section 20 shown in FIGS. 1 and 2A. Moreover, when the connector 25 is detachably connected to a light source device (not shown), the light guide 251 is connected to the light source device. The other constitution may be similar to that of the above embodiment.

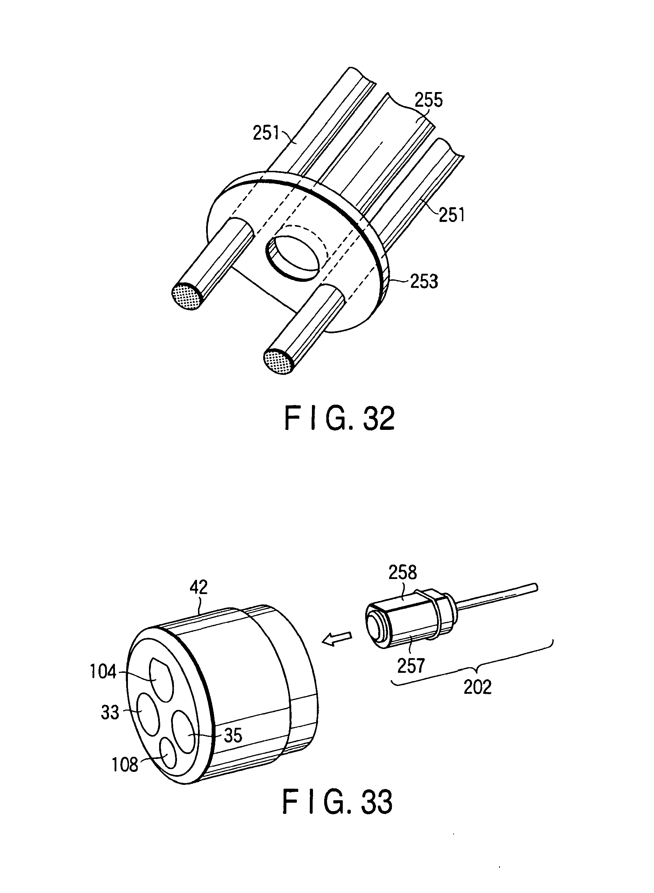

As shown in FIG. 32, the distal end of a guide tube 255 for guiding the only camera section unit 202 is attached to the holding member 253. The holding member 253 is a positioning member for positioning and arranging the distal end opening of the guide tube 255 with respect to a rear end inlet portion 134 of a receiving chamber 132 for receiving the distal end portion of the camera section unit 202. Therefore, when the camera section unit 202 is introduced into the guide tube 255, the distal end portion of the camera section unit 202 is guided to the receiving chamber 132 through the guide tube 255.

The proximal end portion of the guide tube 255 is connected to a branching member 14 at the proximal end of the body cavity insertion section, to communicate with an insertion port 123 provided in the branching member 14. Thus, the guide tube 255 constitutes an insertion guide mechanism for inserting the observation unit 200 to the distal end of the body cavity insertion section 12. Even in this modification, in the same manner as in the above embodiments, there is provided an observation unit attachment/detachment mechanism for detachably attaching the observation unit 200 to the body cavity insertion section 12, a positioning/fixing mechanism for positioning and fixing the observation unit 200 in the body cavity insertion section 12 or the like. Moreover, the distal end portion of the camera section unit 202 may have a round cross-sectional shape. However, when the distal end portion is formed into a flat elliptic shape or the like and the cross-sectional shape of an insertion path of the above insertion guide mechanism is adapted to the shape of the camera section unit 202 to constitute a regulating section for determining the direction of the above observation unit around an axis thereof, the direction of the camera section unit 202 inserted into the insertion guide mechanism is easily determined.

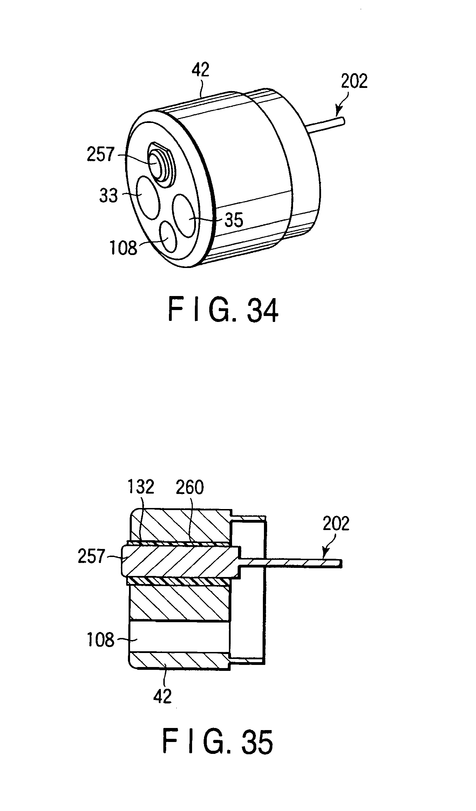

Moreover, as shown in FIG. 33, a part of the outer periphery of the round cross-sectional shape of a distal end chip 257 of the camera section unit 202 is cut to form a flat portion 258. On the other hand, an opening as an observation opening 104 of the second rigid portion 42 is adapted to the shape of the distal end chip 257. In this case, when the distal end chip 257 is received in the receiving chamber 132 as shown in FIG. 34, the direction of the distal end chip 257 around the axis thereof is determined by the flat portion 258.

Next, as shown in FIG. 35, when an elastic body 260 for covering the distal end portion of the camera section unit 202 is provided on the outer periphery of the distal end portion of the unit and the distal end portion of the camera section unit 202 is pushed inwards and received in the receiving chamber 132, the distal end portion of the camera section unit 202 can be fixed to the distal end of the body cavity insertion section 12. Moreover, a portion between the periphery of the distal end portion of the camera section unit 202 and the inner surface of the receiving chamber 132 is sealed to constitute a liquid-tight mechanism which prevents the invasion of a liquid from the outside of the body cavity insertion section 12.

In the above embodiments, a pair of operating arm sections 32, 34 are provided, but three or more operating arm sections may be provided. The present invention is not limited to the above embodiments, and include all embodiments without departing from the scope of the present invention. Moreover, the embodiments may be combined into a constitution.

## Claims

1. An endoscope main body (100) of an endoscope (10) with detachable observation unit comprising:
a body cavity insertion section (12) including a main body bending portion (44) at the distal end portion of the endoscope main body; and further including at least one operating arm section (32, 34) having an arm section bending portion (38, 40), a proximal end portion (40) of the arm section bending portion (38, 40) being connected to the distal end of the body cavity insertion section (12), wherein the operating arm section is configured to be operated and bent;
the endoscope main body further comprising
a main body operating section (20) having a first operation mechanism (23) provided on the proximal end side of the body cavity insertion section to operate and bend the main body bending portion;
an arm section operating section (41) having a second operation mechanism (41) provided on the proximal end side of the body cavity insertion section to operate and bend the arm section bending portion of the operating arm section; and
an observation unit attachment/detachment mechanism (142, 96, 132) which detachably attaches, to the body cavity insertion section, an observation unit (200) provided in the body cavity insertion section and having a camera section (226) and a signal transmission section (228) which transmits video data,
wherein the observation unit attachment/detachment mechanism (142, 96, 132) includes:
an insertion guide mechanism (142, 96, 134) which is formed in the body cavity insertion section (12) and through which the observation unit (200) is inserted to the distal end of the body cavity insertion section; and
an observation unit positioning/fixing mechanism (132 (105; 140)) which positions and fixes, to the body cavity insertion section, the observation unit inserted through the insertion guide mechanism
**characterized in that** the insertion guide mechanism (142, 96, 134) includes a separation member (96) forming an insertion path (97) through which the observation unit (200) is inserted, in the body cavity insertion section (12), separately from internal members arranged in the main body bending portion (44) of the body cavity insertion section,
the separation member (96) is constituted of a tubular member (96) and arranged in the body cavity insertion section (12), and
the tubular member (96) includes a positioning portion (152) which engages with a wire guide (86) to guide a wire (76) which bends the main body bending portion (44) in the body cavity insertion section (12), to position the tubular member in the body cavity insertion section.

2. The endoscope main body (100) of the endoscope (10) with detachable observation unit according to claim 1, **characterized in that** the observation unit positioning/fixing mechanism (132) includes a distal end portion positioning/fixing mechanism (105; 140) which fixes the distal end portion of the observation unit (200) inserted into the body cavity insertion section (12) to a predetermined position by the distal end of the body cavity insertion section.

3. The endoscope main body (100) of the endoscope (10) with detachable observation unit according to claim 2, **characterized in that** the distal end portion positioning/fixing mechanism (105; 140) includes a liquid-tight mechanism (140) which prevents the invasion of a liquid from the outside of the body cavity insertion section to the insertion path (97) at a fixing position where the distal end portion of the observation unit (200) is fixed to the distal end of the body cavity insertion section (12).

4. The endoscope main body (100) of the endoscope (10) with detachable observation unit according to claim 2 or 3, **characterized in that** the proximal end of the body cavity insertion section (12) is provided with a branching portion (14) which branches the main body operating section (20) and the arm section operating section (41), and the body cavity insertion section is connected to the main body operating section via a first extending section (16) extending from the branching portion and connected to the arm section operating section via a second extending section (18) extending from the branching portion.

5. The endoscope main body (100) of the endoscope (10) with detachable observation unit according to claim 4, **characterized in that** the branching portion (14) is provided with an observation unit insertion port (144) of the observation unit attachment/detachment mechanism (142).

6. The endoscope main body (100) of the endoscope (10) with detachable observation unit according to claim 5, **characterized in that** the observation unit insertion port (144) is provided with an insertion guide portion (144) which regulates the inserting direction of the observation unit when inserting the observation unit (200) into the observation unit insertion port.

7. The endoscope main body (100) of the endoscope (10) with detachable observation unit according to any one of claims 1 to 3, **characterized in that** one extending portion (241) extends from the proximal end of the body cavity insertion section (12), and is provided with the main body operating section (20) and the arm section operating section (41).

8. An endoscope (10) with detachable observation unit **characterized by** comprising:
the endoscope main body (100) of the endoscope with detachable observation unit (10) according to any one of claims 1 to 7; and
the observation unit (200),
**characterized in that** the observation unit (200) includes a positioning portion (162) which engages with a wire guide (86) to guide a wire (76) which bends the main body bending portion (44) and which is guided by the wire guide (86).

9. The endoscope (10) with detachable observation unit according to claim 8, **characterized in that** the observation unit (200) includes a camera section unit (202) including a camera section (226) and an illumination section (224), and the camera section unit is connected to a rigid, linear drawing member (160).

10. The endoscope (10) with detachable observation unit according to claim 8 or 9, **characterized in that** the observation unit (200) includes an illumination mechanism (251) which illuminates a view field to be observed.

## Patentansprüche

1. Endoskophauptkörper (100) eines Endoskops (10) mit einer abnehmbaren Beobachtungseinheit; mit:
einem Abschnitt (12) zum Einfügen in einen Körperhohlraum, der ein Hauptkörperbiegestück (44) am distalen Endstück des Endoskophauptkörpers umfasst; und der weiterhin mindestens einen Betätigungsarmabschnitt (32, 34) umfasst, der ein Armabschnittbiegeteil (38, 40) aufweist, wobei ein proximaler Endstück (40) des Armabschnittbiegeteils (38, 40) mit dem distalen Ende des Abschnitts (12) zum Einführen in den Körperhohlraum verbunden ist, wodurch der Betätigungsarmabschnitt dazu aufgebaut ist, betätigt und gebogen zu werden;
wobei der Endoskophauptkörper weiterhin Folgendes umfasst:
einen Hauptkörperbetätigungsabschnitt (20), der einen ersten Betätigungsmechanismus (23) umfasst, der an der proximalen Endseite des Abschnitts zum Einführen in den Körperhohlraum vorgesehen ist, um das Hauptkörperbiegestück zu betätigen und zu biegen;
einem Armabschnittbetätigungsabschnitt (41), der einen zweiten Betätigungsmechanismus (41) umfasst, der an der proximalen Endseite des Abschnitts zum Einführen in den Körperhohlraum vorgesehen ist, um das Armabschnittbiegeteil des Betätigungsarmabschnitts zu betätigen und zu biegen; und
einen Mechanismus (142, 96, 132) zum Anbringen/Lösen einer Beobachtungseinheit, der lösbar am Abschnitt zum Einführen in den Körperhohlraum eine Beobachtungseinheit (200) anbringt, die in dem Abschnitt zum Einführen in den Körperhohlraum vorgesehen ist und einen Kameraabschnitt (226) und einen Signalübertragungsabschnitt (228) zum Übertragen von Videodaten aufweist"
wobei der Mechanismus (142, 96, 132) zum Anbringen/Lösen der Beobachtungseinheit Folgendes umfasst:
einen Einführungsleitmechanismus (142, 96, 134), der in dem Abschnitt (12) zum Einführen in den Körperhohlraum gebildet ist und durch den die Beobachtungseinheit (200) in das distale Ende des Abschnitts zum Einführen in den Körperhohlraum eingeführt ist; und
einen Mechanismus (132 (105; 140)) zum Positionieren/Fixieren der Beobachtungseinheit, der die Beobachtungseinheit, die durch den Einführungsleitmechanismus eingeführt ist, an dem Abschnitt zum Einführen in den Körperhohlraum positioniert und fixiert,
**dadurch gekennzeichnet, dass** der Einführungsleitmechanismus (142, 96, 134) ein Trennungsteil (96) umfasst, das einen Einfügeweg (97) bildet, durch den die Beobachtungseinheit (200) in den Abschnitt zum Einführen in den Körperhohlraum getrennt von internen Teilen eingefügt ist, die in dem Hauptkörperbiegeabschnitt (44) des Abschnitts zum Einführen in den Körperhohlraum angeordnet sind,
das Trennungsteil (96) aus einem röhrenförmigen Teil (96) gebildet ist und in dem Abschnitt (12) zum Einführen in den Körperhohlraum angeordnet ist, und
das röhrenförmige Teil (96) einen Positionierabschnitt (152) aufweist, der mit einer Drahtführung (86) zusammenwirkt, um einen Draht (76) zu führen, der den Hauptkörperbiegeabschnitt (44) in dem Abschnitt (12) zum Einführen in den Körperhohlraum biegt, um das röhrenförmige Teil in dem Abschnitt zum Einführen in den Körperhohlraum zu positionieren.

2. Endoskophauptkörper (100) des Endoskops (10) mit abnehmbarer Beobachtungseinheit nach Anspruch 1, **dadurch gekennzeichnet, dass** der Mechanismus (132) zum Positionieren/Fixieren der Beobachtungseinheit einen Mechanismus (105; 140) zum Positionieren/Fixieren des distalen Endstücks umfasst, der den distalen Endstück der Beobachtungseinheit (200), der in den Abschnitt (12) zum Einführen in den Körperhohlraum eingeführt ist, durch das distale Ende des Abschnitts zum Einführen in den Körperhohlraum an einer vorab festgelegten Position fixiert.

3. Endoskophauptkörper (100) des Endoskops (10) mit abnehmbarer Beobachtungseinheit nach Anspruch 2, **dadurch gekennzeichnet, dass** der Mechanismus (105; 140) zum Positionieren/Fixieren des distalen Endstücks einen fluiddichten Mechanismus (140) umfasst, der das Eintreten eines Fluids von außerhalb des Abschnitts zum Einführen in den Körperhohlraum in den Einführweg (97) an einer Befestigungsposition verhindert, an der der distale Endstück der Beobachtungseinheit (200) am distalen Ende des Abschnitts (12) zum Einführen in den Körperhohlraum befestigt ist.

4. Endoskophauptkörper (100) des Endoskops (10) mit abnehmbarer Beobachtungseinheit nach Anspruch 2 oder 3, **dadurch gekennzeichnet, dass** das proximale Ende des Abschnitts (12) zum Einführen in den Körperhohlraum mit einem Verzweigungsabschnitt (14) versehen ist, der den Hauptkörperbetätigungsabschnitt (20) und den Armabschnittbetätigungsabschnitt (41) verzweigt, und der Abschnitt zum Einfügen in den Körperhohlraum ist mit dem Hauptkörperbetätigungsabschnitt über einen ersten Erstreckungsabschnitt (16) verbunden, der sich vom Verzweigungsabschnitt weg erstreckt und mit dem Armabschnittbetätigungsabschnitt über einen zweiten Erstreckungsabschnitt (18) verbunden ist, der sich vom Verzweigungsabschnitt weg erstreckt.

5. Endoskophauptkörper (100) des Endoskops (10) mit abnehmbarer Beobachtungseinheit nach Anspruch 4, **dadurch gekennzeichnet, dass** der Verzweigungsabschnitt (14) mit einem Anschluss (144) zum Einführen der Beobachtungseinheit des Mechanismus (142) zum Anbringen/Lösen der Beobachtungseinheit versehen ist.

6. Endoskophauptkörper (100) des Endoskops (10) mit abnehmbarer Beobachtungseinheit nach Anspruch 5, **dadurch gekennzeichnet, dass** der Anschluss (144) zum Einführen der Beobachtungseinheit ein Einführungsleitstück (144) umfasst, das die Einführrichtung der Beobachtungseinheit beim Einführen der Beobachtungseinheit (200) in den Anschluss zum Einführen der Beobachtungseinheit reguliert.

7. Endoskophauptkörper (100) des Endoskops (10) mit abnehmbarer Beobachtungseinheit nach einem der Ansprüche 1 bis 3, **dadurch gekennzeichnet, dass** ein Erstreckungsabschnitt (241) sich vom proximalen Ende des Abschnitts (12) zum Einführen in den Körperhohlraum erstreckt und mit dem Hauptkörperbetätigungsabschnitt (20) und dem Armabschnittbetätigungsabschnitt (41) versehen ist.

8. Endoskop (10) mit einer abnehmbaren Beobachtungseinheit, **dadurch gekennzeichnet, dass** es Folgendes umfasst:
den Endoskophauptkörper (100) des Endoskops (10) mit abnehmbarer Beobachtungseinheit nach einem der Ansprüche 1 bis 7; und
die Beobachtungseinheit (200),
**dadurch gekennzeichnet, dass** die Beobachtungseinheit (200) einen Positionierungsabschnitt (162) umfasst, der mit einen Führungsdraht (86) zusammenwirkt, um einen Draht (76) zu führen, der den Hauptkörperbiegeabschnitt (44) biegt und der durch den Führungsdraht (86) geführt ist.

9. Endoskop (10) mit einer abnehmbaren Beobachtungseinheit nach Anspruch 8, **dadurch gekennzeichnet, dass** die Beobachtungseinheit (200) eine Kameraabschnittseinheit (202) umfasst, die einen Kameraabschnitt (226) und einen Beleuchtungsabschnitt (224) aufweist, und wobei der Kameraabschnitt mit einem festen, linearen Zugteil (160) verbunden ist.

10. Endoskop (10) mit einer abnehmbarem Beobachtungseinheit nach Anspruch 8 oder 9, **dadurch gekennzeichnet, dass** die Beobachtungseinheit (200) einen Beleuchtungsmechanismus (251) umfasst, der ein zu beobachtendes Gesichtsfeld beleuchtet.

## Revendications

1. Corps principal d'endoscope (100) d'un endoscope (10) à unité d'observation détachable, comprenant :
une section d'insertion de cavité de corps (12) incluant une partie de flexion de corps principal (44) au niveau de la partie d'extrémité distale du corps principal d'endoscope ; et incluant en outre au moins une section formant bras de commande (32, 34) comportant une partie de flexion de section formant bras (38, 40), une partie d'extrémité proximale (40) de la partie de flexion de section formant bras (38, 40) étant reliée à l'extrémité distale de la section d'insertion de cavité de corps (12), dans lequel la section formant bras de commande est configurée pour être commandée et fléchie ;
le corps principal d'endoscope comprenant en outre
une section de commande de corps principal (20) comportant un premier mécanisme de commande (23) disposé sur le côté d'extrémité proximale de la section d'insertion de cavité de corps pour commander et fléchir la partie de flexion du corps principal ;
une section de commande de section formant bras (41) comportant un second mécanisme de commande (41) disposé sur le côté d'extrémité proximale de la section d'insertion de cavité de corps pour commander et fléchir la partie de flexion de la section formant bras de la section formant bras de commande ; et
un mécanisme d'attachement/de détachement de l'unité d'observation (142, 96, 132) qui attache de manière détachable, à la section d'insertion de cavité de corps, une unité d'observation (200) disposée dans la section d'insertion de cavité de corps et comportant une section caméra (226) et une section de transmission de signal (228) qui transmet des données vidéo,
dans lequel le mécanisme d'attachement/de détachement de l'unité d'observation (142, 96, 132) inclut :
un mécanisme de guidage d'insertion (142, 96, 134) qui est formé dans la section d'insertion de cavité de corps (12) et à travers lequel l'unité d'observation (200) est insérée jusqu'à l'extrémité distale de la section d'insertion de cavité de corps ; et
un mécanisme de positionnement/fixation d'unité d'observation (132 (105 ; 140)) qui positionne et fixe, sur la section d'insertion de cavité de corps, l'unité d'observation insérée à travers le mécanisme de guidage d'insertion ;
**caractérisé en ce que** le mécanisme de guidage d'insertion (142, 96, 134) inclut un élément de séparation (96) formant un trajet d'insertion (97) à travers lequel l'unité d'observation (200) est insérée, dans la section d'insertion de cavité de corps (12), séparément des éléments internes disposés dans la partie de flexion du corps principal (44) de la section d'insertion de cavité de corps,
l'élément de séparation (96) est constitué d'un élément tubulaire (96) et disposé dans la section d'insertion de cavité de corps (12), et
l'élément tubulaire (96) inclut une partie de positionnement (152) qui se met en prise avec un passe-fil (86) pour guider un fil (76) qui fléchit la partie de flexion du corps principal (44) dans la section d'insertion de cavité de corps (12), pour positionneur l'élément tubulaire dans la section d'insertion de cavité de corps.

2. Corps principal d'endoscope (100) de l'endoscope (10) à unité d'observation détachable selon la revendication 1, **caractérisé en ce que** le mécanisme de positionnement/fixation (132) de l'unité d'observation inclut un mécanisme de positionnement/fixation de partie d'extrémité distale (105 ; 140) qui fixe la partie d'extrémité distale de l'unité d'observation (200) insérée dans la section d'insertion de cavité de corps (12) à une position prédéterminée par l'extrémité distale de la section d'insertion de cavité de corps.

3. Corps principal d'endoscope (100) de l'endoscope (10) à unité d'observation détachable selon la revendication 2, **caractérisé en ce que** le mécanisme de positionnement/fixation de la partie d'extrémité distale (105 ; 140) inclut un mécanisme étanche au liquide (140) qui empêche la pénétration d'un liquide depuis l'extérieur de la section d'insertion de cavité de corps vers le trajet d'insertion (97), au niveau d'une position de fixation sur laquelle la partie d'extrémité distale de l'unité d'observation (200) est fixée à l'extrémité distale de la section d'insertion de cavité de corps (12).

4. Corps principal d'endoscope (100) de l'endoscope (10) à unité d'observation détachable selon la revendication 2 ou 3, **caractérisé en ce que** l'extrémité proximale de la section d'insertion de cavité de corps (12) est dotée d'une partie branchement (14) qui branche la section de commande du corps principal (20) et la section de commande de section formant bras (41), et la section d'insertion de cavité de corps est reliée à la section de commande du corps principal par le biais d'une première section d'extension (16) s'étendant à partir de la partie branchement et reliée à la section de commande de la section formant bras par le biais d'une seconde section d'extension (18) s'étendant à partir de la partie branchement.

5. Corps principal d'endoscope (100) de l'endoscope (10) à unité d'observation détachable selon la revendication 4, **caractérisé en ce que** la partie branchement (14) est dotée d'un orifice d'insertion de l'unité d'observation (144) du mécanisme d'attachement/détachement (142) de l'unité d'observation.

6. Corps principal d'endoscope (100) de l'endoscope (10) à unité d'observation détachable selon la revendication 5, **caractérisé en ce que** l'orifice d'insertion de l'unité d'observation (144) est doté d'une partie formant guide d'insertion (144) qui régule la direction d'insertion de l'unité d'observation lors de l'insertion de l'unité d'observation (200) dans l'orifice d'insertion de l'unité d'observation.

7. Corps principal d'endoscope (100) de l'endoscope (10) à unité d'observation détachable selon l'une quelconque des revendications 1 à 3, **caractérisé en ce qu'**une partie d'extension (241) s`étend de l'extrémité proximale de la section d'insertion de cavité de corps (12), et est dotée de la section de commande de corps principal (20) et de la section de commande de la section formant bras (41).

8. Endoscope (10) à unité d'observation détachable, **caractérisé en ce qu'**il comprend :
le corps principal d'endoscope (100) de l'endoscope à unité d'observation détachable (10) selon l'une quelconque des revendications 1 à 7 ; et
l'unité d' observation (200),
**caractérisé en ce que** l'unité d'observation (200) inclut une partie positionnement (162) qui se met en prise avec un passe-fil (86) pour guider un fil (76) qui fléchit la partie de flexion du corps principal (44) et qui est guidé par le passe-fil (86).

9. Endoscope (10) à unité d'observation détachable selon la revendication 8, **caractérisé en ce que** l'unité d'observation (200) inclut une unité de section formant caméra (202) incluant une section formant caméra (226), et une section d'éclairage (224), et l'unité de section formant caméra est reliée à un élément de traction linéaire rigide (160).

10. Endoscope (10) à unité d'observation détachable selon la revendication 8 ou 9, **caractérisé en ce que** l'unité d'observation (200) inclus un mécanisme d'éclairage (251) qui éclaire un champ de vision à observer.
